# EUROPEAN PATENT SPECIFICATION

(11) **EP 4 125 728 B1**
(45) Date of publication and mention of the grant of the patent: **08.04.2026**
(21) Application number: 21730736.2
(22) Date of filing: 14.05.2021
(51) Int. Cl.: A61F 2/24

(54) **PROSTHETIC HEART VALVE LEAFLET COMMISSURE ASSEMBLIES AND METHOD OF ASSEMBLING**
PROTHETISCHE HERZKLAPPENSEGELKOMMISSURANORDNUNGEN UND MONTAGEVERFAHREN
ENSEMBLES D'ASSEMBLAGE DE COMMISSURES DE FEUILLETS DE VALVES CARDIAQUES PROTHÉTIQUES ET MÉTHODE D'ASSEMBLAGE

(30) Priority: 14.05.2020 US 202063024951 P
(43) Date of publication of application: 08.02.2023
(73) Proprietor: Edwards Lifesciences Corporation, Irvine, CA 92614-5688 (US)
(72) Inventor: ZAMANI, Shahram, Irvine, CA 92614 (US); O'DELL, Tyler Dale, Irvine, CA 92614 (US); LEVI, Tamir S., 30889 Caesarea (IL); BUKIN, Michael, 30889 Caesarea (IL); NIR, Noam, 30889 Caesarea (IL); YOHANAN, Ziv, 30889 Caesarea (IL)
(74) Representative: Venner, Julia Ann
(86) International application number: PCT/US2021/032387
(87) International publication number: WO 2021/231824

(56) References cited:
- WO-A1-2012/106011
- US-A1- 2018 028 310
- US-A1- 2020 069 415

## Description

### CROSS REFERENCE TO RELATED APPLICATION

This application claims the benefit of U.S. Provisional Patent Application No. 63/024,951, filed May 14, 2020.

### FIELD

The present disclosure relates to prosthetic heart valves, and to methods and assemblies for forming commissures with leaflets of such prosthetic heart valves.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery device and advanced through the patient's vasculature (e.g., through a femoral artery and the aorta) until the prosthetic valve reaches the implantation site in the heart. The prosthetic valve is then expanded to its functional size, for example, by inflating a balloon on which the prosthetic valve is mounted, actuating a mechanical actuator that applies an expansion force to the prosthetic valve, or by deploying the prosthetic valve from a sheath of the delivery device so that the prosthetic valve can self-expand to its functional size.

Prosthetic valves that rely on a mechanical actuator for expansion can be referred to as "mechanically expandable" prosthetic heart valves. The actuator typically takes the form of pull cables, sutures, wires and/or shafts that are configured to transmit expansion forces from a handle of the delivery apparatus to the prosthetic valve.

Most expandable, transcatheter heart valves comprise a cylindrical metal frame or stent and prosthetic leaflets mounted inside the frame. The leaflets may be attached to the frame at commissure tabs (also referred to as leaflet tabs) of the leaflets. For example, a commissure may be formed by connecting the commissure tabs of two adjacent leaflets to one another, and in some embodiments, to a commissure support (or attachment) element configured to couple to a commissure support portion of the frame. The commissure or the commissure support element can then be attached to the commissure support portion of the frame via a fastener, such as a suture.

Typical commissures or commissure assemblies can be relatively complex and time consuming to form and suture to the commissure support portion of the frame, in part due to the numerous stitches that can be required. Further, these types of commissures and attachment methods to the commissure support portion can be subject to wear along the numerous stitches, as well as along portions of the commissure tabs that wrap around a side and/or outer-facing surface of the commissure support portion. In some examples, the mounted commissure can deteriorate due to displacement of the commissure out of its initial, secured position, including rotating around the commissure support portion and sliding axially, up and down, along the commissure support portion. Further, in some embodiments, vertical folding of the commissure tabs (e.g., in an axial direction relative to a central longitudinal axis of the frame) to form commissures can result in a less robust attachment to the commissure support portion of the frame.

Frames of prosthetic heart valves, in general, go through foreshortening and therefore the leaflets undergo changes during the crimping and expansion of the frame of the valve. Leaflets of the prosthetic heart valve can also experience stress from cyclical forces experienced during operation of the prosthetic heart valve, *in vivo.* For example, the repeated opening and closing of the leaflet assembly of the valve may cause degradation to the leaflets, such as degradation to a tissue collagen matric of bovine or porcine tissue that makes up the leaflets.

Accordingly, a need exists for improved prosthetic heart valve leaflet assemblies, and commissures formed with such leaflet assemblies, and methods for assembling commissures to a frame of a prosthetic heart valve.

US 2018/0028310 describes a prosthetic heart valve including a radially collapsible and expandable annular frame having a plurality of struts defining openings. The prosthetic heart valve can further include a plurality of leaflets that regulate the flow of blood through the frame. Projecting from the upper corners of the leaflet are integral upper tabs, also known as commissure tabs. To assemble the commissure, each upper tab is folded along a horizontal fold line to form first and second tab layers. The first and second tab layers can then be folded lengthwise along a vertical fold line to form an outer folded portion and an inner folded portion. The outer folded portion can be secured to a commissure attachment member with stitches that extend through the first and second tab layers and the commissure attachment member.

### SUMMARY

The prosthetic heart valve and the method of assembling according to the invention are defined in the independent claims. Particular embodiments of the invention are defined in the dependent claims. Any subject matter contained herein that does not fall within the scope of the appended claims is considered as being useful for understanding the invention.

Described herein are embodiments of prosthetic heart valves and methods for assembling prosthetic heart valves including an annular frame and leaflet assembly. In some embodiments, commissures can be formed by folding a pair of commissure tabs of adjacent leaflets in the leaflet assembly such that the folded portions of the pair of commissure tabs are arranged in a radial direction and securing the folded pair of commissure tabs to a protruding portion of an attachment member that extends outward from a base portion of the attachment member. The base portion of the attachment member can then be secured to a commissure support portion of the frame in order to secure the commissure to the frame.

A first aspect of the presently claimed invention provides a prosthetic heart valve including: an annular frame comprising a plurality of commissure support portions and a plurality of leaflets situated within the frame, each leaflet comprising a body and two opposing commissure tabs arranged on opposite sides of the body, each commissure tab being paired with an adjacent commissure tab of an adjacent leaflet to form a commissure. Each commissure of the prosthetic heart valve includes an attachment member including a first portion, the first portion extending across and attached directly to a corresponding commissure support portion of the plurality of commissure support portions or to a commissure support element configured to be coupled to the commissure support portion, and a second portion that protrudes radially outward from the first portion and extends radially inward toward a central longitudinal axis of the frame. Each commissure further includes a first commissure tab of a first leaflet of the plurality of leaflets folded into two overlapping layers that overlap in a lateral direction, the lateral direction arranged perpendicular to an axial direction and a radial direction that are relative to the central longitudinal axis, the two overlapping layers arranged adjacent to a first side of the second portion of the attachment member and secured directly to the attachment member. Each commissure further includes a second commissure tab of a second leaflet of the plurality of leaflets folded into two overlapping layers that overlap in the lateral direction, the two overlapping layers arranged adjacent to an opposite, second side of the second portion of the attachment member and secured directly to the attachment member.

A second aspect of the presently claimed invention provides a method of assembling a prosthetic heart valve comprising a plurality of leaflets including forming a plurality of commissures with the plurality of leaflets, each leaflet including two opposing commissure tabs arranged on opposite sides of a body of the leaflet. Each commissure is formed by: folding each of a first commissure tab of a first leaflet and a second commissure tab of an adjacent, second leaflet into two overlapping layers that overlap in a lateral direction, the lateral direction arranged perpendicular to an axial direction and a radial direction that are relative to a central longitudinal axis of an annular frame of the prosthetic heart valve; arranging the folded first commissure tab against a first side of a protruding portion of an attachment member and arranging the folded second commissure tab against an opposite, second side of the protruding portion, the protruding portion extending in the radial direction, away from a base portion of the attachment member; securing each of the first commissure tab and the second commissure tab to the attachment member; and, for each commissure, attaching the attachment member to a respective commissure support portion of the annular frame, either directly or via a commissure support element configured to be coupled to the commissure support portion, the base portion of the attachment member arranged between, in the radial direction, the folded first and second commissure tabs and the commissure support portion or the commissure support element.

In another representative embodiment according to the first aspect of the presently claimed invention, a prosthetic heart valve includes: an annular frame comprising a plurality of commissure support portions, each commissure support portion including an inner surface facing a central longitudinal axis of the frame and an oppositely arranged, outer surface; and a plurality of leaflets situated within the frame, each leaflet comprising a body and two opposing commissure tabs arranged on opposite sides of the body, each commissure tab being paired with an adjacent commissure tab of an adjacent leaflet to form a commissure. Each commissure of the prosthetic heart valve includes an attachment member attached directly to a corresponding commissure support portion of the plurality of commissure support portions, the attachment member including a base portion extending across the inner surface of the commissure support portion and a protruding portion that extends radially outward from a central region of the base portion and extends radially inward toward the central longitudinal axis. Each commissure further includes a first commissure tab of a first leaflet of the plurality of leaflets folded into two overlapping layers that overlap in a lateral direction, the lateral direction arranged perpendicular to an axial direction and a radial direction that are relative to the central longitudinal axis, the two overlapping layers arranged adjacent to a first side of the protruding portion of the attachment member and secured directly to the attachment member. Each commissure further includes a second commissure tab of a second leaflet of the plurality of leaflets folded into two overlapping layers that overlap in the lateral direction, the two overlapping layers arranged adjacent to an opposite, second side of the protruding portion of the attachment member and secured directly to the attachment member.

Also disclosed herein is a prosthetic heart valve including: an annular frame comprising a plurality of commissure support portions; a plurality of leaflets situated within the frame, each leaflet comprising a body and two opposing commissure tabs arranged on opposite sides of the body, each commissure tab being paired with an adjacent commissure tab of an adjacent leaflet to form a commissure; and a plurality of commissure support elements, each commissure support element including a coupling portion coupled to a corresponding one of the plurality of commissure support portions and a commissure-receiving portion. Each commissure of the prosthetic heart valve includes an attachment member attached directly to a corresponding commissure support element, the attachment member including a base portion extending across an inner surface of the commissure-receiving portion of the commissure support element and a protruding portion that extends radially outward from a central region of the base portion and extends radially inward toward a central longitudinal axis of the frame. Each commissure further includes a first commissure tab of a first leaflet of the plurality of leaflets folded into two overlapping layers that overlap in a lateral direction, the lateral direction arranged perpendicular to an axial direction and a radial direction that are relative to the central longitudinal axis, the two overlapping layers arranged adjacent to a first side of the protruding portion of the attachment member and secured directly to the attachment member. Each commissure further includes a second commissure tab of a second leaflet of the plurality of leaflets folded into two overlapping layers that overlap in the lateral direction, the two overlapping layers arranged adjacent to an opposite, second side of the protruding portion of the attachment member and secured directly to the attachment member.

Also disclosed herein is a prosthetic heart valve including: an annular frame comprising a plurality of interconnected and angled struts defining a plurality of rows of cells arranged between an outflow end and an inflow end of the frame and a plurality of leaflets situated within the frame, each leaflet comprising a body and two opposing commissure tabs arranged on opposite sides of the body, each commissure tab being paired with an adjacent commissure tab of an adjacent leaflet to form a commissure. Each commissure of the prosthetic heart valve includes an attachment member attached directly to angled struts defining one cell included in the plurality of rows of cells, the attachment member including a base portion extending across the cell and a protruding portion that extends radially outward from a central region of the base portion and extends radially inward toward the central longitudinal axis. Each commissure further includes a first commissure tab of a first leaflet of the plurality of leaflets folded into two overlapping layers that overlap in a lateral direction, the lateral direction arranged perpendicular to an axial direction and a radial direction that are relative to the central longitudinal axis, the two overlapping layers arranged adjacent to a first side of the protruding portion of the attachment member and secured directly to the attachment member. Each commissure further includes a second commissure tab of a second leaflet of the plurality of leaflets folded into two overlapping layers that overlap in the lateral direction, the two overlapping layers arranged adjacent to an opposite, second side of the protruding portion of the attachment member and secured directly to the attachment member.

Also disclosed herein is a prosthetic heart valve including: an annular frame comprising a plurality of interconnected and angled struts defining a plurality of rows of cells arranged between an outflow end and an inflow end of the frame and a plurality of commissure support portion and a plurality of leaflets situated within the frame, each leaflet comprising a body and two opposing commissure tabs arranged on opposite sides of the body, the body including an outflow edge extending across the leaflet, between the two opposing commissure tabs, and cusp edge portions that meet to form an inflow end portion of the leaflet. Each commissure tab is paired with an adjacent commissure tab of an adjacent leaflet to form a commissure, where each commissure tab includes one or more stitching lines comprising one or more apertures adapted to receive a suture, where each stitching line extends between an upper edge and a lower edge of the commissure tab and includes a portion that is offset from a remainder of the stitching line, toward the body of the leaflet, the portion including one or more apertures that are arranged inward of a first outer aperture of the stitching line that is arranged adjacent to the upper edge, the upper edge connected to the outflow edge of the leaflet. Each commissure is secured directly to a corresponding commissure support portion of the plurality of commissure support portions or to an attachment member configured to be coupled to the commissure support portion, via one or more sutures extending along the one or more stitching lines.

Also disclosed herein is a prosthetic heart valve comprising an annular frame comprising a plurality of commissure support portions and a plurality of leaflets situated within the frame, each leaflet comprising a body and two opposing commissure tabs arranged on opposite sides of the body, each commissure tab being paired with an adjacent commissure tab of an adjacent leaflet to form a commissure. Each commissure of the prosthetic heart valve comprises an attachment member including a first portion, the first portion extending across and attached directly to a corresponding commissure support portion of the plurality of commissure support portions or to a commissure support element configured to be coupled to the commissure support portion, and a second portion that protrudes radially outward from the first portion and extends radially inward toward a central longitudinal axis of the frame; a first commissure tab of a first leaflet of the plurality of leaflets arranged adjacent to a first side of the second portion of the attachment member and secured directly to the attachment member; and a second commissure tab of a second leaflet of the plurality of leaflets arranged adjacent to an opposite, second side of the second portion of the attachment member and secured directly to the attachment member.

The foregoing and other objects, features, and advantages of the invention will become more apparent from the following detailed description, which proceeds with reference to the accompanying figures.

### BRIEF DESCRIPTION OF THE DRAWINGS

FIG. 1 is a perspective view of an exemplary embodiment of a prosthetic heart valve.
FIG. 2 is a perspective view of a frame for a prosthetic heart valve comprising three expansion and locking mechanisms, according to another embodiment.
FIG. 3 is a top view of the frame and expansion and locking mechanisms of FIG. 2.
FIG. 4 is a side view of a frame for a prosthetic heart valve having a tapered shape, according to an embodiment.
FIG. 5 is a side view of another embodiment of a frame of a prosthetic heart valve, the frame having a tapered shape.
FIG. 6 is a plan view of an exemplary leaflet for a prosthetic heart valve.
FIG. 7 is a cross-sectional, top view of an embodiment of a commissure of a prosthetic heart valve in which the commissure is secured to a support post of the prosthetic heart valve.
FIG. 8 is a front, perspective view of an inner side of the support post of FIG. 7, with the commissure of FIG. 7 secured to the support post.
FIG. 9 is a back, perspective view of an outer side of the support post of FIG. 7, with the commissure of FIG. 7 secured to the support post.
FIG. 10 is a perspective view of a prosthetic heart valve including a frame with open commissure windows, according to an embodiment.
FIG. 11 is a perspective view of an exemplary frame of a prosthetic heart valve including open commissure windows.
FIG. 12 is a perspective view of an embodiment of a commissure support element and a corresponding actuator component of a frame of a prosthetic heart valve adapted to receive the commissure support element.
FIG. 13 is a perspective view of the embodiment of the commissure support element of FIG. 12 coupled to the corresponding actuator component.
FIG. 14 is a cross-sectional, top view of the embodiment of the commissure of FIG. 7, in which the commissure is secured to an open commissure window of a frame of a prosthetic heart valve or a commissure support element configured to be coupled to the frame.
FIG. 15 is a detail, perspective view of the embodiment of the commissure of FIG. 7, in which the commissure is secured to a cell of a frame of a prosthetic heart valve.
FIG. 16 is a side view of an exemplary prosthetic heart valve including the commissures of FIG. 15.
FIG. 17 is flow chart of a method for assembling a prosthetic heart valve comprising a plurality of leaflets.
FIG. 18 is a detail view of a portion of an exemplary leaflet with a first embodiment of a stitching line, at commissure tabs of the leaflet, that is relatively straight.
FIG. 19 is a detail view of a portion of an exemplary leaflet with a second embodiment of a stitching line, at the commissure tabs, with a portion that is offset from a remainder of the stitching line.
FIG. 20 is a detail view of a portion of an exemplary leaflet with a third embodiment of a stitching line, at the commissure tabs, with a portion that is offset from a remainder of the stitching line.
FIG. 21 is a side view of an embodiment of a transcatheter delivery apparatus for delivering a prosthetic heart valve to a target implantation site, with the prosthetic heart valve retained in a radially compressed state within a capsule of the delivery apparatus.
FIG. 22 is a side view of the transcatheter delivery apparatus of FIG. 21, with the capsule retracted to uncover the prosthetic heart valve.

### DETAILED DESCRIPTION

### General Considerations

For purposes of this description, certain aspects, advantages, and novel features of the embodiments of this disclosure are described herein. The described methods, systems, and apparatus should not be construed as limiting in any way. Instead, the present disclosure is directed toward all novel and non-obvious features and aspects of the various disclosed embodiments, alone and in various combinations and sub-combinations with one another. The disclosed methods, systems, and apparatus are not limited to any specific aspect, feature, or combination thereof, nor do the disclosed methods, systems, and apparatus require that any one or more specific advantages be present, or problems be solved. The scope of the invention is defined by the claims.

Features, integers, characteristics, compounds, chemical moieties, or groups described in conjunction with a particular aspect, embodiment or example of the disclosure are to be understood to be applicable to any other aspect, embodiment or example described herein unless incompatible therewith. All of the features disclosed in this specification (including any accompanying claims, abstract, and drawings), and/or all of the steps of any method or process so disclosed, may be combined in any combination, except combinations where at least some of such features and/or steps are mutually exclusive.

Although the operations of some of the disclosed methods are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods, systems, and apparatus can be used in conjunction with other systems, methods, and apparatus.

As used herein, the terms "a," "an," and "at least one" encompass one or more of the specified element. That is, if two of a particular element are present, one of these elements is also present and thus "an" element is present. The terms "a plurality of" and "plural" mean two or more of the specified element.

As used herein, the term "and/or" used between the last two of a list of elements means any one or more of the listed elements. For example, the phrase "A, B, and/or C" means "A," "B," "C," "A and B," "A and C," "B and C," or "A, B, and C."

As used herein, the term "coupled" generally means physically coupled or linked and does not exclude the presence of intermediate elements between the coupled items absent specific contrary language.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same. As used herein, "and/or" means "and" or "or," as well as "and" and "or."

As used herein, with reference to the prosthetic heart valve and the delivery apparatus, "proximal" refers to a position, direction, or portion of a component that is closer to the user and/or a handle of the delivery apparatus that is outside the patient, while "distal" refers to a position, direction, or portion of a component that is further away from the user and/or the handle of the delivery apparatus and closer to the implantation site. The terms "longitudinal" and "axial" refer to an axis extending in the proximal and distal directions, unless otherwise expressly defined. Further, the term "radial" refers to a direction that is arranged perpendicular to the axis and points along a radius from a center of an object (where the axis is positioned at the center, such as the longitudinal axis of the prosthetic valve).

### Examples of the Disclosed Technology

Described herein are examples of prosthetic heart valves, leaflet assemblies, and commissures for prosthetic heart valves, and methods for assembling commissures of prosthetic heart valves. The prosthetic heart valves may include an annular frame and a plurality of leaflets attached to the frame via commissures formed by joining pairs of adjacent ends (referred to herein as commissure tabs) of the leaflets. The formation of the commissures may include folding each of a first commissure tab of a first leaflet and a second commissure tab of an adjacent, second leaflet into two overlapping layers that overlap in a lateral direction, the lateral direction arranged perpendicular to an axial direction and a radial direction that are relative to a central longitudinal axis of the annular frame. The formation of the commissure may further include arranging the folded first commissure tab against a first side of a protruding portion of an attachment member and arranging the folded second commissure tab against an opposite, second side of the protruding portion, the protruding portion extending in the radial direction, away from a base portion of the attachment member. In some embodiments, the attachment member can be a flexible fabric or polymeric material. The formation of the commissure may further include securing each of the first commissure tab and the second commissure tab to the attachment member and then attaching the attachment member to a respective commissure support portion of the frame, either directly or via a commissure support element configured to be coupled to the commissure support portion.

In some embodiments, the base portion of the attachment member can be arranged between, in the radial direction, the folded first and second commissure tabs and the commissure support portion or the commissure support element. As a result, the commissure tabs of the leaflets can be blocked from coming into contact with the frame. This commissure configuration may reduce stresses on the leaflets during valve operation and during radial expansion and/or contraction of the frame, thereby increasing their longevity.

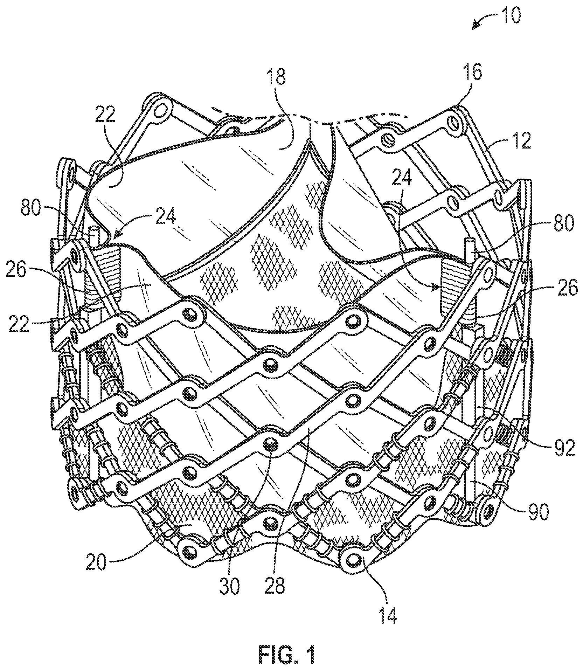

FIG. 1 shows an exemplary prosthetic heart valve 10, according to one embodiment. The prosthetic heart valve 10 can be radially compressible and expandable between a radially compressed configuration for delivery into a patient and a radially expanded configuration (as shown in FIG. 1). In particular embodiments, the prosthetic heart valve 10 can be implanted within the native aortic annulus, although it also can be implanted at other locations in the heart, including within the native mitral valve, the native pulmonary valve, and the native tricuspid valve. The prosthetic heart valve 10 can include an annular stent or frame 12 having a first end 14 and a second end 16.

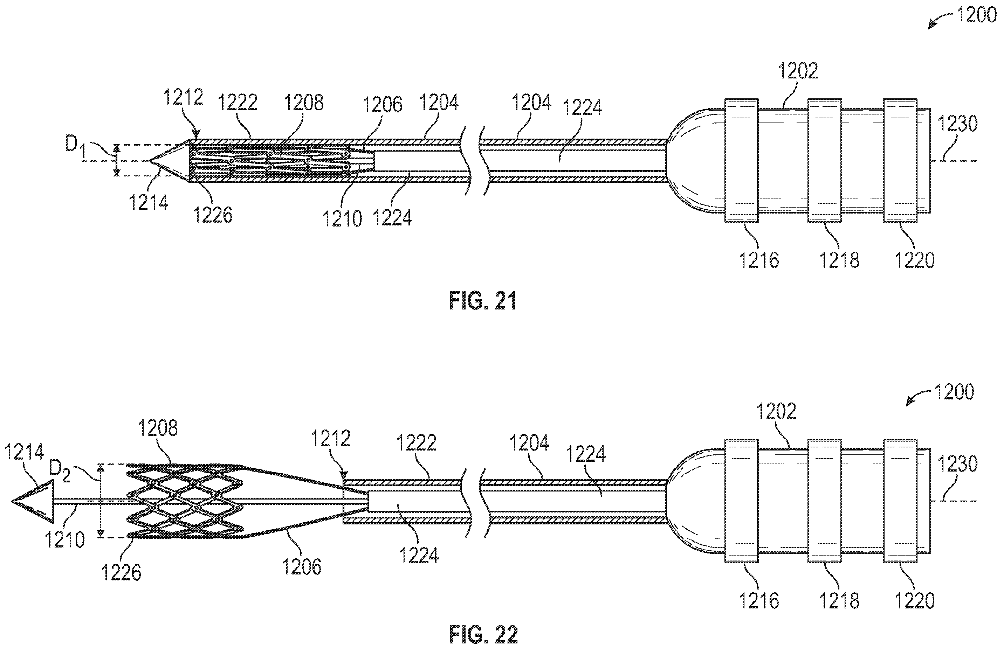

In the depicted embodiment, the first end 14 is an inflow end and the second end 16 is an outflow end. The outflow end 16 can be coupled to a delivery apparatus for delivering and implanting the prosthetic heart valve within the native aortic valve in a transfemoral, retrograde delivery approach. Thus, in the delivery configuration of the prosthetic heart valve, the outflow end 16 is the proximal-most end of the prosthetic valve. In other embodiments, the inflow end 14 can be coupled to the delivery apparatus, depending on the particular native valve being replaced and the delivery technique that is used (e.g., trans-septal, transapical, etc.). For example, the inflow end 14 can be coupled to the delivery apparatus (and therefore is the proximal-most end of the prosthetic heart valve in the delivery configuration) when delivering the prosthetic heart valve to the native mitral valve via a trans-septal delivery approach. An exemplary delivery apparatus that can be used to deliver the prosthetic heart valve is shown in FIGS. 21 and 22 and described in more detail below.

Returning to FIG. 1, the frame 12 can be made of any of various suitable materials, such as stainless steel, a cobalt chromium alloy, or a nickel titanium alloy ("NiTi"), for example Nitinol. Referring again to FIG. 1, as shown, the frame 12 can include a plurality of interconnected struts 28 arranged in a lattice-type pattern. The struts 28 are shown as positioned diagonally, or offset at an angle relative to, and radially offset from, a longitudinal axis of the prosthetic heart valve 10 when the prosthetic heart valve 10 is in the expanded configuration. In other implementations, the struts 28 can be offset by a different amount than depicted in FIG. 1, or some or all of the struts 28 can be positioned parallel to the longitudinal axis of the prosthetic heart valve 10.

In the illustrated embodiment, the struts 28 are pivotably coupled to one another at one or more pivot joints along the length of each strut. For example, in the illustrated configuration, each of the struts 28 can be formed with apertures at opposing ends of the strut and apertures spaced along the length of the strut. Respective hinges can be formed at the locations where struts 28 overlap each other via fasteners or pivot members, such as rivets or pins 30 that extend through the apertures. The hinges can allow the struts 28 to pivot relative to one another as the frame 12 is radially expanded or compressed, such as during assembly, preparation, or implantation of the prosthetic heart valve 10.

In some embodiments, the frame 12 can be constructed by forming individual components (e.g., the struts and fasteners of the frame) and then mechanically assembling and connecting the individual components together. In other embodiments, the struts 28 are not coupled to each other with respective hinges but are otherwise pivotable or bendable relative to each other to permit radial expansion and contraction of the frame 12. For example, the frame 12 can be formed (e.g., via laser cutting, electroforming or physical vapor deposition) from a single piece of material (e.g., a metal tube). Further details regarding the construction of the frame and the prosthetic heart valve are described in U.S. Patent Application Publication Nos. 2018/0153689, 2018/0344456, and 2019/0060057.

The prosthetic heart valve 10 can also include a valvular structure 18 which is coupled to the frame 12 and configured to regulate the flow of blood through the prosthetic heart valve 10 from the inflow end 14 to the outflow end 16. The prosthetic heart valve 10 can further include a plurality of actuators 80 mounted to and equally spaced around the inner surface of the frame 12. The actuators are configured to apply expansion and compression to the frame for radially expanding and compressing the prosthetic valve.

In the illustrated embodiment, the actuators 80 are linear actuators, each of which comprises an inner member, or piston, 90 and an outer member, or cylinder, 92. The inner member 90 is pivotably coupled to a junction of the frame, such as at the first end 14, while the outer member 92 is pivotably coupled to another junction of the frame closer to the second end 16. Moving the inner member 90 proximally relative to the outer member 92 and/or moving the outer member 92 distally relative to the inner member 90 is effective to radially expand the prosthetic valve. Conversely, moving the inner member 90 distally relative to the outer member 92 and/or moving the outer member 92 proximally relative to the inner member 90 is effective to radially compress the prosthetic valve. The actuators 80 can include locking mechanisms that are configured to retain the prosthetic valve in an expanded state inside the patient's body.

In some embodiments, each of the actuators 80 can be configured to form a releasable connection with one or more respective actuators of a delivery apparatus of a transcatheter delivery system. The actuators of the delivery apparatus can transmit forces from a handle of the delivery apparatus to the actuators 80 for expanding or compressing the prosthetic valve. Further details of the actuators, locking mechanisms and delivery apparatuses for actuating the actuators can be found in U.S. Patent Application Publication Nos. 2018/0153689, 2019/0060057 and 2018/0325665.

Any of the actuators and locking mechanisms disclosed in the previously filed applications can be incorporated in any of the prosthetic valves disclosed herein. Further, any of the delivery apparatuses disclosed in the previously filed applications can be used to deliver and implant any of the prosthetic valves discloses herein.

In some embodiments, each of the actuators 80 can be used to support a respective commissure 24 (described below). As such, the actuators 80 can include commissure support portions for supporting and attaching commissures 24 of the valvular structure 18 to the frame 12, as described further herein.

The valvular structure 18 can include, for example, a leaflet assembly comprising one or more leaflets 22 (three leaflets 22 in the illustrated embodiment) made of a flexible material. The leaflets 22 of the leaflet assembly can be made from in whole or part, biological material, bio-compatible synthetic materials, or other such materials. Suitable biological material can include, for example, bovine pericardium (or pericardium from other sources). Each leaflet 22 includes two opposing commissure tabs arranged on opposite sides of a body of the leaflet. The body of the leaflet may be the portion of the leaflet that is adapted to bend and move during operation of the prosthetic heart valve 10. The commissure tabs of adjacent leaflets 22 can be arranged to form commissures 24, which can be, for example, mounted to commissure support portions of respective actuators 80. Further details regarding transcatheter prosthetic heart valves, including the manner in which the valvular structure can be coupled to the frame 12 of the prosthetic heart valve 10, can be found, for example, in U.S. Patent Nos. 6,730,118, 7,393,360, 7,510,575, 7,993,394, and 8,652,202, and U.S. Patent Application Publication No. 2018/0325665.

In some embodiments, as shown in FIG. 1, the commissures 24 can be mounted (e.g., sutured) directly to commissure support portions of the actuators 80 of the frame 12 via commissure attachments 26. As one example, the commissure attachments 26 may include one or more stitches securing the commissures 24 to corresponding actuators 80. In other embodiments, the commissures 24 can be mounted to support struts or posts of the frame that are separate from the actuators 80. In still other embodiments, the commissures may be secured to an additional commissure attachment or support member (as described further herein) and the support member is then secured to a commissure support portion of an actuator 80 or support struts or posts of the frame.

The prosthetic heart valve 10 can also include one or more skirts or sealing members. For example, as shown in FIG. 1, the prosthetic heart valve 10 can include an inner skirt 20 mounted on the inner surface of the frame 12. As shown in FIG. 1, the inner skirt 20 is a circumferential inner skirt that spans an entire circumference of the inner surface of the frame 12. The inner skirt 20 can function as a sealing member to prevent or decrease perivalvular leakage (e.g., when the valve is placed at the implantation site) and as an attachment surface to anchor the leaflets 22 to the frame 12. For example, the inflow (cusp) edges of the leaflets 22 can be sutured directly to the inner skirt 20, which in turn can be directly connected to selected struts 28 of the frame, such as with sutures, as shown in FIG. 1.

The prosthetic heart valve 10 can also include an outer skirt mounted on the outer surface of the frame 12 (not shown in FIG. 1). The outer skirt can function as a sealing member for the prosthetic valve by sealing against the tissue of the native valve annulus and helping to reduce paravalvular leakage past the prosthetic valve. The inner and outer skirts can be formed from any of various suitable biocompatible materials, including any of various synthetic materials (e.g., PET) or natural tissue (e.g., pericardial tissue). The inner and outer skirts can be mounted to the frame using sutures, an adhesive, welding, and/or other means for attaching the skirts to the frame.

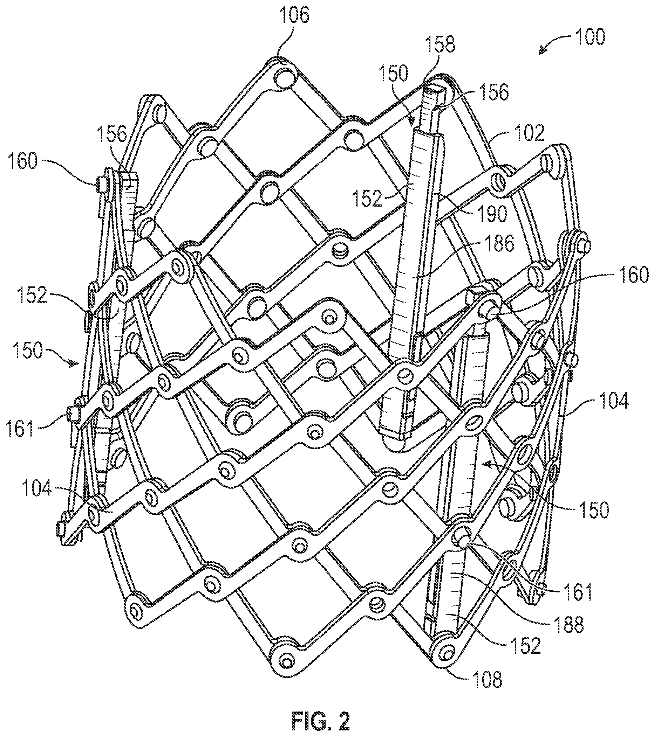

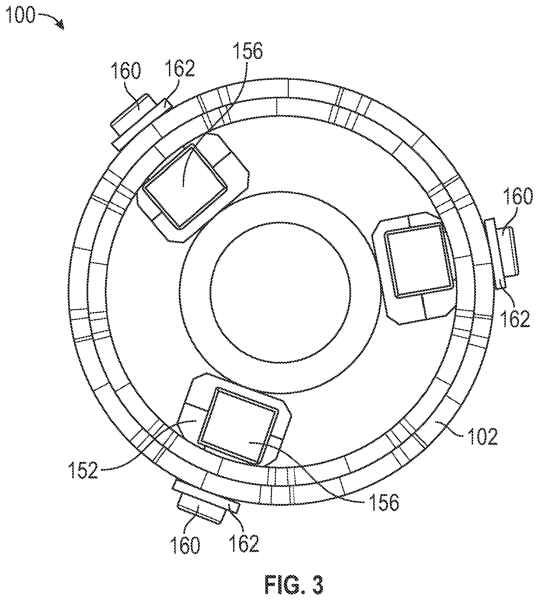

FIGS. 2 and 3 illustrate an exemplary embodiment of a prosthetic valve 100, according to another embodiment, the prosthetic valve 100 comprising a frame 102 and one or more expansion and locking mechanisms 150. The frame 102 comprises a plurality of pivotably connected struts 104 defining an inflow end 106 (which is the distal end of the frame in a delivery configuration for the illustrated embodiment) and an outflow end 108 (which is the proximal end of the frame in the delivery configuration for the illustrated embodiment). The struts 104 are pivotably connected to each other at a plurality of junctions that permit pivoting of the struts relative to each other when the frame 102 is radially compressed and expanded, as described above in connection with prosthetic valve 10.

The prosthetic valve 100 can include a valvular structure (e.g., valvular structure 18) and inner and/or outer skirts, as previously described, although these components are omitted from FIGS. 2 and 3 for purposes of illustration. The one or more expansion and locking mechanisms 150 can be used in lieu of or in addition to actuators 80 described above. The expansion and locking mechanisms 150 can be used to both radially expand and lock the frame 102 of prosthetic valve 100 in a radially expanded state. In some embodiments, the commissures of the leaflets may be attached to a commissure support portion of the expansion and locking mechanisms 150. In alternate embodiments, the commissures of the leaflets may be attached to additional commissure posts of the frame 102.

FIG. 2 shows three expansion and locking mechanisms 150 mounted to the frame 102 with the frame 102 shown in the radially expanded configuration. Though the illustrated embodiment shows three expansion and locking mechanisms 150 spaced apart from each other about the circumference of the frame, it should be noted that a prosthetic valve can comprise any number of expansion and locking mechanisms 150. For example, in some embodiments, a prosthetic valve can comprise a single expansion and locking mechanism, or two expansion and locking mechanisms, or four expansion and locking mechanisms, etc. The expansion and locking mechanisms 150 can be placed at any position about the circumference of the frame 102. For example, in some embodiments, such as the illustrated embodiment, the expansion and locking mechanisms 150 are equally spaced from one another about the circumference of the frame 102. In other embodiments, it can be advantageous to have two or more expansion and locking mechanisms situated adjacent to one another.

Each expansion and locking mechanism 150 can include an outer member in the form of a sleeve 152 having an inner lumen, cavity, or bore and an inner member 156 extending at least partially into the cavity. The sleeve 152 in the illustrated embodiment comprises an inner wall 186, an outer wall 188, and two side walls 190, each of which extends radially between a longitudinal edge of the inner wall 186 and an opposing longitudinal edge of the outer wall 188. The inner wall 186, the outer wall 188, and the two side walls 190 define the cavity, which is sized and shaped to receive the inner member 156.

The sleeve 152 in the illustrated embodiment has a rectangular shape in cross-section and the inner member 156 has a rectangular shape in cross-section corresponding to the shape of the bore. In other embodiments, the sleeve 152 and/or the inner member 156 can have a square cross-sectional profile. As shown in FIG. 3, the rectangular and/or square cross-sections can advantageously minimize the distance that the expansion and locking members extend into the lumen of the frame 102, which can reduce the overall crimp profile of the valve 100. However, in other embodiments, the sleeve and the inner member can have any of various corresponding shapes in cross-section, for example, circular, ovular, triangular, rectangular, square, or combinations thereof.

As best shown in FIG. 1, a distal end portion 158 of the inner member 156 can be coupled to the frame 102 at a first location via a fastener 160 that is affixed to and extends radially from the distal end portion 158 of the inner member 156. The fastener 160 can be for example, a rivet or pin. As shown, in some embodiments, the fastener 160 can extend through corresponding apertures at a junction of two overlapping struts 104 of the frame 102 and can serve as a pivot pin around which the two struts 104 can pivot relative to each other and the inner member 156. In some embodiments, an end cap or nut 162 (as shown in FIG. 3) can be disposed over an end portion of the fastener 160. The nut 162 can have a diameter greater than the diameter of the apertures to retain the fastener 160 within the apertures. In alternative embodiments, the inner member 156 need not comprise a fastener 160 and can be coupled to the frame 102 via other means of attachment such as welding, adhesives, etc.

The sleeve 152 can be coupled to the frame 102 at a second location, axially spaced from the first location. For example, in the illustrated embodiment, the inner member 156 is secured to the frame 102 near the distal or inflow end 106 of the frame and the sleeve 152 is secured to the frame 102 closer to or at the proximal or outflow end 108 of the frame, such as via a fastener 161 (e.g., a rivet or pint). The fastener 161 is affixed to and extends radially from the sleeve 152 through corresponding apertures at a junction of two overlapping struts 104 and can serve as a pivot pin around which the two struts 104 can pivot relative to each other and the sleeve 152. A nut 162 can be mounted on each fastener 161 to retain the fastener within the corresponding apertures. The expansion and locking mechanism 150 can be pivotably coupled to the frame 102 at any two axially spaced, circumferentially aligned locations on the frame.

The inner member 156 can be axially movable relative to the sleeve 152 in a proximal direction and in a distal direction, along a central longitudinal axis of the frame 102. As such, because the inner member 156 and the sleeve 152 are secured to the frame at axially spaced locations, moving the inner member 156 and the sleeve 152 axially with respect to one another in a telescoping manner can cause radial expansion or compression of the frame 102. For example, moving the inner member 156 proximally toward the outflow end 108 of the frame, while holding the sleeve 152 in a fixed position and/or moving the sleeve 152 distally toward the inflow end 106 of the frame can cause the frame 102 to foreshorten axially and expand radially. Conversely, moving the inner member 156 distally and/or moving the sleeve 152 proximally causes the frame 102 to elongate axially and compress radially.

A prosthetic valve 100 including one or more expansion and locking mechanisms 150 can be expanded in the following exemplary manner. Generally, the prosthetic valve 100 is placed in a radially compressed state and releasably coupled to a distal end portion of a delivery apparatus, and then advanced through the vasculature of a patient to a selected implantation site (e.g., the native aortic annulus). The prosthetic valve 100 can then be deployed at the implantation site and expanded and locked in the expanded configuration using the expansion and locking mechanisms 150. Further details regarding the prosthetic valve, the expansion and locking mechanisms, and delivery apparatuses for actuating the expansion and locking mechanism can be found in International Application Pub. No. WO2021/086933 A2.

FIGS. 21 and 22 illustrate a delivery apparatus 1200, according to one embodiment, adapted to deliver a prosthetic heart valve (or prosthetic valve) 1208, such as the prosthetic heart valve 10 illustrated in FIG. 1 and/or the prosthetic valve 100 illustrated in FIGS. 2-3, as described above, to a target implantation site in a patient. FIGS. 21-22 show the prosthetic valve 1208 in different configurations relative to the delivery apparatus 1200 during a valve implantation procedure. The prosthetic valve 1208 can be releasably coupled to one or more components of the delivery apparatus 1200, as described above and further below. It should be understood that the delivery apparatus 1200 can be used to implant prosthetic devices other than prosthetic valves, such as stents or grafts.

The delivery apparatus 1200 in the illustrated embodiment generally includes a handle 1202, an elongate shaft 1204 (which comprises an outer, or outermost, shaft in the illustrated embodiment) extending distally from the handle 1202, an inner (e.g., innermost) shaft 1210, an intermediate shaft 1224 arranged coaxial with and between (in the radial direction which is perpendicular to a central longitudinal axis 1230 of the delivery apparatus 1200) the outer shaft 1204 and the inner shaft 1210, and at least one actuator assembly (e.g., member or actuator) 1206 for expanding and compressing the prosthetic valve 1208, the at least one actuator assembly 1206 extending through the outer shaft 1204 and distally outwardly from a distal end portion 1212 of the outer shaft 1204.

In some embodiments, the outer shaft 1204, inner shaft 1210, intermediate shaft 1224, and/or actuator assembly 1206 may make up a delivery apparatus catheter of the delivery apparatus 1200, controlled by and attached to the handle 1202.

The delivery apparatus 1200 can include three actuator assemblies 1206 (only two of the three are shown in FIGS. 21-22) releasably coupled to the prosthetic valve 1208. However, in alternate embodiments, the delivery apparatus 1200 may include more or less than three actuator assemblies 1206 (e.g., one, two, four, or the like). As shown in FIGS. 21-22, the plurality of actuator assemblies 1206 are circumferentially spaced apart from each other around a circumference of the delivery apparatus 1200 and can extend axially through the outer shaft 1204 from the handle 1202 to the prosthetic valve 1208.

In particular embodiments, each actuator assembly 1206 can be releasably coupled to a corresponding actuator of the prosthetic valve (e.g., an actuator 80 as shown in FIG. 1 or a portion of an expansion and locking mechanism 150 as shown in FIGS. 2 and 3). Each actuator assembly 1206 can include an inner member having a distal end releasably coupled to an inner member of an actuator of the prosthetic valve and an outer member having a distal end releasably coupled to an outer member of an actuator of the prosthetic valve. In another embodiment, each actuator assembly 1206 can be an actuator assembly releasably coupled to the prosthetic valve 1208 via a threaded sleeve.

In some embodiments, the intermediate shaft 1224 may be adapted to house and organize the actuator assemblies 1206. For example, the actuator assemblies 1206 may be housed within and extend outwardly from a distal end of the intermediate shaft 1224. In some embodiments, each actuator assembly 1206 may be kept separate from the other actuator assemblies 1206 within the intermediate shaft 1224. For example, each actuator assembly 1206 can extend through a separate lumen of the intermediate shaft 1224.

As shown in FIGS. 21-22, a distal end of the inner shaft 1210 may include a nosecone 1214 which may be used to guide the delivery apparatus 1200 through a lumen of a patient to a target implantation site for the prosthetic valve 1208. The nosecone 1214 may be arranged proximate to a distal end 1226 of the prosthetic valve 1208.

In use, the delivery apparatus 1200 can be releasably coupled to the prosthetic valve 1208 to produce radial expansion and compression of the frame of the prosthetic valve 1208. In some embodiments, the actuator assemblies 1206 of the delivery apparatus 1200 can be configured to transfer pushing and/or pulling forces from the handle 1202 of the delivery apparatus 1200 to the prosthetic valve 1208. For example, in some embodiments, the actuator assemblies 1206 may have distal end portions that can be releasably connected to the prosthetic valve 1208 via respective release-and-locking units.

In some embodiments, the outer shaft 1204 of the delivery apparatus 1200 can be configured as a steerable guide catheter having an adjustable curvature for use in steering the delivery apparatus 1200 through the patient's vasculature. In particular embodiments, the outer shaft 1204 can include a steerable distal section, the curvature of which can be adjusted by the operator to assist in guiding the apparatus through the patient's vasculature.

The outer shaft 1204 and the actuator assemblies 1206 can be moved relative to one another (axially and/or rotationally) to facilitate delivery and positioning of the prosthetic valve 1208 at an implantation site in the patient's body.

In some embodiments, the distal end portion 1212 of the outer shaft 1204 can form and/or function as a sheath (or capsule) 1222 that is sized and shaped to receive and house the prosthetic valve 1208 in a radially compressed state for delivery into and through a patient's vasculature. Once the prosthetic valve 1208 is advanced to the implantation site or adjacent the implantation site, the prosthetic valve 1208 can be advanced from the capsule 1222 by retracting the outer shaft 1204, and thus the capsule 1222, axially, along central longitudinal axis 1230, relative to the actuator assemblies 1206 and the prosthetic valve 1208. As such, the prosthetic valve 1208 may be uncovered while the capsule 1222 moves axially back towards the handle 1202 (e.g., in a proximal direction along the central longitudinal axis 1230). In alternative embodiments, the prosthetic valve 1208 can be advanced from the capsule 1222 by advancing the actuator assemblies 1206 relative to the outer shaft 1204, after which the prosthetic valve 1208 can be radially expanded.

The advancement of the prosthetic valve 1208 from the sheath by axially moving the actuator assemblies 1206 relative to the outer shaft 1204 or by retracting the outer shaft 1204 relative to the actuator assemblies 1206 may be actuated by operating a first knob 1216 on the handle 1202. The first knob 1216 can be operatively connected to a proximal end portion of the outer shaft 1204 and can be configured to retract the outer shaft 1204 proximally relative to the actuator assemblies 1206 to deploy the prosthetic valve 1208 from the distal end portion 1212 of the capsule 1222 or operatively connected to proximal ends of the actuator assemblies 1206 to advance the actuator assemblies 1206 distally relative to the outer shaft 1204 to deploy the prosthetic valve 1208 from the distal end portion 1212 of the capsule 1222. The first knob 1216 may be a slidable or rotatable adjustment element that is operatively connected to the actuator assemblies 1206 and/or the outer shaft 1204.

The handle 1202 may include additional adjustment knobs, such as a second knob 1218 and a third knob 1220, as shown in FIGS. 21-22. In some embodiments, the second knob 1218 may be operatively coupled to the actuator assemblies 1206 and actuate the actuator assemblies 1206 to adjust the prosthetic valve 1208 from a non-expanded (or radially compressed) configuration (as shown in FIG. 21) to a radially expanded configuration, and vice versa.

In some embodiments, the third knob 1220 may be operatively coupled to the actuator assemblies 1206 and actuate the actuator assemblies 1206 to disconnect from the prosthetic valve 1208. As a result, the prosthetic valve 1208 may be detached from the delivery apparatus 1200 and implanted (deployed) at the target implantation site.

FIG. 21 shows the prosthetic valve 1208 retained in a radially compressed state within the capsule 1222 of the delivery apparatus 1200. As such, in FIG. 21, the prosthetic valve 1208 is in its radially compressed configuration having a smallest diameter, D1. The smallest diameter D1 may be approximately the same as an inner diameter of the capsule 1222. The capsule 1222 surrounding an outside of the prosthetic valve 1208, as shown in FIG. 21, may maintain the prosthetic valve in the radially compressed configuration. As a result, the prosthetic valve 1208 may be advanced through a patient's vasculature, for example, to the target implantation site via the delivery apparatus 1200.

After reaching the target implantation site, the capsule 1222 may be pulled (or retracted) away from the nosecone 1214 and the prosthetic valve 1208, in a proximal direction along the central longitudinal axis 1230 of the delivery apparatus 1200, to uncover the prosthetic valve 1208. In alternate embodiments, the actuator assemblies 1206 may be advanced, in the distal direction, to move the prosthetic valve 1208 out of the capsule 1222.

FIG. 22 shows the prosthetic valve 1208 in the uncovered (unsheathed) state where it is arranged outside of the capsule 1222. At this state, the prosthetic valve 1208 is not actively expanded via the actuator assemblies 1206. However, since it is no longer bound by (e.g., retained within) the capsule 1222, the prosthetic valve 1208 may assume a partially expanded diameter D2 which is larger than the smallest diameter D1 due to the inherent resiliency of the struts of the frame. It should be noted that the extent of expansion of the prosthetic valve 1208, from the compressed, smallest diameter D1 (FIG. 21) to the partially expanded diameter D2 (FIG. 22) may be exaggerated in FIG. 22 for the purposes of illustration. After being deployed from the capsule 1222, as shown in FIG. 22, the prosthetic valve 1208 can then be radially expanded and implanted, by actuation of the actuator assemblies 1206.

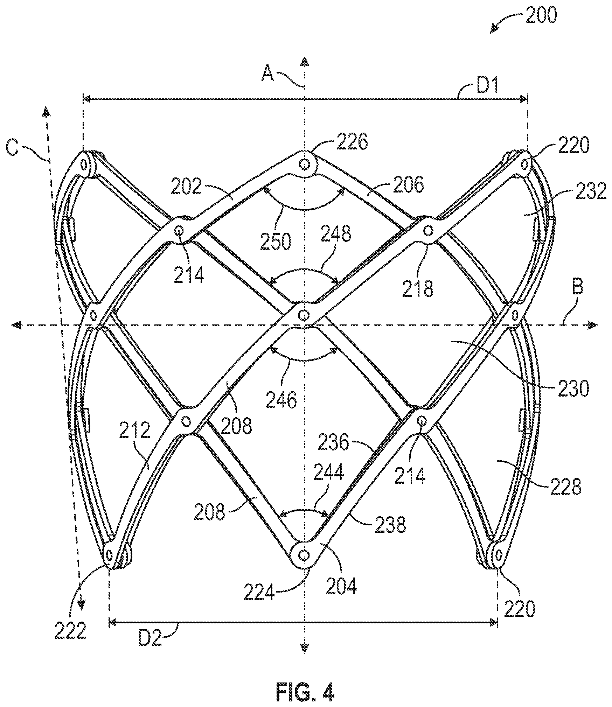

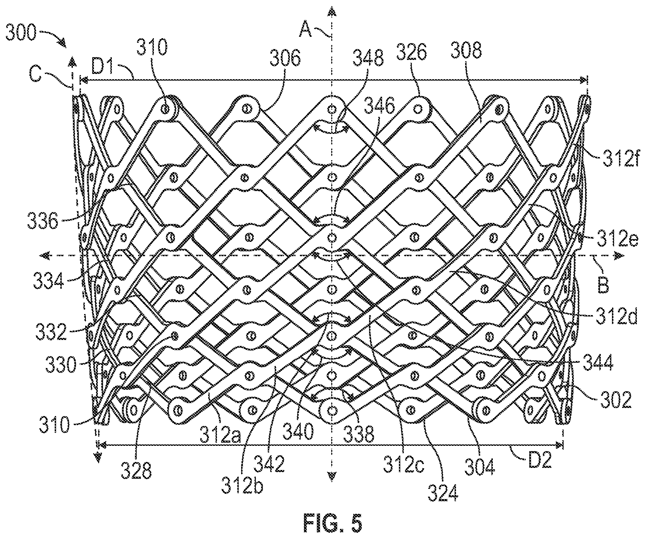

Turning now to the embodiments shown in FIGS. 4 and 5, in some embodiments, a frame of a prosthetic heart valve can comprise struts that are shaped to form a non-cylindrical shape when expanded. For example, such frames can be tapered, along an axial direction relative to a central longitudinal axis of the frame. In some embodiments, the frame can be tapered from the outflow end to the inflow end.

FIG. 4 illustrates another embodiment of a prosthetic valve 200 comprising a frame 202 shown in its deployed, radially expanded configuration. The prosthetic valve 200 can include a valvular structure (e.g., valvular structure 18), inner and/or outer skirts, and actuators (e.g., actuators 80 of FIG. 1) or expansion and locking mechanism 150 of FIGS. 2-3), as previously described, although these components are omitted from FIG. 4 for purposes of illustration. The frame 202 can have an inflow end portion 204 defining an inflow end 224 of the frame 202 and an outflow end portion 206 defining an outflow end 226 of the frame 202. The prosthetic valve 200 can define a longitudinal axis (central longitudinal axis) A extending from the inflow end portion 204 to the outflow end portion 206 and a lateral axis B extending perpendicular to the longitudinal axis A. While only one side of the frame 202 is depicted in FIG. 4, it should be appreciated that frame 202 forms an annular structure having an opposite side that is identical to the portion shown.

The frame 202 comprises a plurality of interconnected struts 208 arranged in a lattice-type pattern. Each strut can fully extend from the inflow end 224 of the frame 202 to the outflow end 226 of the frame. Thus, in the illustrated embodiment, the frame 202 can be formed entirely from struts that extend continuously from the inflow end 224 to the outflow end 226. In alternative embodiments, the frame 202 can have struts that are connected end-to-end along the length of the frame.

Each of the struts 208 can include a plurality of apertures that can be spaced unequally along the length of each strut 208, defining a plurality of segments 212 having unequal lengths. In the illustrated embodiment, the segments 212 of each strut 208 can decrease in length from the inflow end portion 204 to the outflow end portion 206 of the frame 202. In the assembled frame 202, the struts 208 form a plurality of closed cells arranged in a plurality of circumferentially extending rows of cells with the cells becoming progressively smaller from the inflow end 224 to the outflow end 226. In the illustrated embodiment, each strut 208 has five apertures defining four segments 112 and three rows of cells, including a first row of cells 228, a second row of cells 230, and a third row of cells 232 with the cells 228 being the largest, the cells 230 being smaller than the cells 228 and the cells 232 being smaller than the cells 230.

In other embodiments, each strut can have a greater or fewer number of apertures to define a different number of strut segments and rows of frame cells. For example, FIG. 5 shows a prosthetic valve 300 (described below) wherein each strut comprises seven apertures.

As shown in FIG. 4, the varying lengths of the strut segments also form angles 244, 246, 248, 250 between pivotably connected struts wherein the angles progressively increase from the inflow end 224 to the outflow end 226. In alternative embodiments, one or more segments can have unequal lengths and one or more segments can have equal lengths.

As shown in FIG. 4, each strut 208 can be curved helically with respect to the longitudinal axis A of the frame 202 to define an annular shape of the frame 202. The helical curve provides each strut 208 with a concave, radial inner surface (the surface facing the longitudinal axis A) and an opposing convex, radial outer surface (the surface facing away from the longitudinal axis A).

The apertures can be used to connect the struts 208 to one another using fasteners 214, such as those described above with reference to prosthetic valve 10 (FIG. 1). In other embodiments, the apertures 210 and/or fasteners 214 can be omitted. For example, the struts 208 can be fixedly connected to one another, such as by welding or adhesion, or by laser-cutting the individual struts of the frame from a metal tube.

As shown, the segments 212 can be arranged end-to-end relative to each other with adjacent ends interconnected to each other by intermediate segments 218. The struts 208 can have enlarged (relative to segments 212) end portions 220 that form the apices 222 at the inflow and outflow ends 224, 226 of the frame 202. Each of the intermediate segments 218 and end portions 220 can have a respective aperture, such as at its geometric center, for receiving a fastener 214. Each segment 212 can be slightly laterally offset from an adjacent segment 212 in a direction perpendicular to the overall length of the strut 208, as shown. In alternative embodiments, the segments 212 can be arranged without any offset relative to each other.

In the illustrated embodiment, each segment 212 of the strut 208 is curved such that the overall shape of the strut 208 is curved with respect to the lateral axis B (or any line parallel to axis B and perpendicular to axis A).

In particular embodiments, each strut 208 can have a continuous and constant curve from one end of the strut to the other end of the strut. In other embodiments, the projection of each segment 212 in a plane parallel to the longitudinal axis A can be straight (i.e., each segment 112 is straight except for any helical curvature with respect to the longitudinal axis A) and the amount of offset of each segment 112 relative to an adjacent segment 112 along the length of strut 208 can vary such that the overall shape of the strut 208 is curved along its length with respect to the lateral axis B (or any line parallel to axis B and perpendicular to axis A); that is, a line extending from one end of the strut to the other end and intersecting each segment 212 is curved with respect to axis B. Alternatively, individual strut segments 212 can be straight and connected end-to-end to each other at non-zero angles such that the overall shape of the strut 208 is curved along its length with respect to the lateral axis B (or any line parallel to axis B and perpendicular to axis A). In other embodiments, one or more of the struts of a frame can have a non-constant or variable curvature along its length (in which case the center of curvature of the strut can vary as one moves along the length of the strut).

As shown in FIG. 4, each strut 208 can be curved and arranged such that it is convex with respect to the outflow end 226 of the frame 202. As such, each strut 208 in the illustrated embodiment has a convex, first longitudinal edge 236 facing the outflow end 226 of the frame and a concave, second longitudinal edge 238 facing the inflow end 224 of the frame. Due to the unique shape of the struts 208, the frame 202 formed by the struts has a non-Euclidian geometry, and in particular, an elliptic geometry (also referred to as Riemannian geometry).

As shown in FIG. 4, in the expanded configuration, the curvature of the struts 208 can give the frame 202 a non-cylindrical, tapered shape (e.g., a frustoconical shape, a V-shape, or a Y-shape) wherein the outflow end 226 has a first diameter D1 larger than a second diameter D2 of the inflow end 224. The degree of taper can be referred to as the draft angle of the frame 202, which can be a measure of the angle between the longitudinal axis A and a line C drawn tangent to the outer surface of the frame. When implanted within the native annulus of a patient, the larger outflow relative to the inflow created by the tapered shape can reduce the pressure gradient across the prosthetic valve, helping to improve hemodynamics and mitigate the risk of paravalvular leakage.

In particular embodiments, the draft angle between lines A and C can be at least 2 degrees, at least 5 degrees, at least 10 degrees, at least 20 degrees, at least 30 degrees, at least 40 degrees, or at least 50 degrees. In particular embodiments, the draft angle can be between 2 and 15 degrees. In particular embodiments, the ratio of the outflow diameter D1 to the inflow diameter D2 is at least greater than 1, at least greater than 1.1, at least greater than 1.2, at least greater than 1.3, at least greater than 1.4, or at least greater than 1.5.

In some embodiments, there is a 2-3 mm difference between the outflow diameter D1 and the inflow diameter D2. In one specific example, the outflow diameter D1 is about 30 mm and the inflow diameter D2 is about 27 mm. In another example, the outflow diameter D1 is about 31.5 mm and the inflow diameter D2 is about 29 mm. In another example, the outflow diameter D1 is about 24.5 mm and the inflow diameter D2 is about 22 mm.

In some embodiments, while in the crimped or radially compressed configuration, the frame 202 can retain a tapered shape wherein the outflow end 226 has a diameter larger than a diameter of the inflow end 224 and the draft angle of the frame in the compressed configuration can be greater than the draft angle of the frame when the frame is in the expanded configuration.

FIG. 5 illustrates another embodiment of a prosthetic valve 300 comprising a frame 302 having a tapered shape, shown in its deployed, radially expanded configuration. In particular, the frame 302 has a positive draft angle (e.g., the diameter at the outflow end 326 of the prosthetic valve 300 is greater than the diameter at the inflow end 324), thereby causing the frame 302 to taper from the outflow end 326 to the inflow end 324.

The prosthetic valve 300 is similar to the prosthetic valve 200 except that the prosthetic valve 300 has a frame 302 where each strut 308 comprises seven apertures 310 and therefore has more strut segments and frame cells than the struts of prosthetic valve 200. The frame 302 may be similar to frame 12 of FIG. 1 and frame 102 of FIGS. 2-3, since each strut of these frames also comprises seven apertures.

Similar to prosthetic valve 10 and/or prosthetic valve 100, the prosthetic valve 300 can include a valvular structure (e.g., valvular structure 18), inner and/or outer skirts, and actuators (e.g., actuators 80 of FIG. 1) or expansion and locking mechanisms (e.g., expansion and locking mechanisms 150 of FIGS. 2-3) as previously described, although these components are omitted for purposes of illustration. The frame 302 can have an inflow end portion 304 defining an inflow end 324 of the frame and an outflow end portion 306 defining an outflow end 326 of the frame. The prosthetic valve can define a longitudinal axis A extending from the inflow end portion 304 to the outflow end portion 306 and a lateral axis B extending perpendicular to the longitudinal axis A.

The frame 302 comprises a plurality of interconnected struts 308 which extend from the inflow end 324 to the outflow end 326 of the frame 302. Thus, in the illustrated embodiment, the frame 302 can be formed entirely from struts that extend continuously from the inflow end 324 to the outflow end 326. In alternative embodiments, the frame 302 can have struts that are connected end-to end along the length of the frame.

Each of the struts 308 can include a plurality of apertures 310. As shown, the apertures 310 can be spaced unequally along the length of the strut 308, defining a plurality of segments 312 having unequal lengths. In the illustrated embodiment, the strut 308 comprises segments 312a, 312b, 312c, 312d, 312e, and 312f, with segment 312a being the shortest, and each subsequent segment 312b, 312c, 312d, 312e, and 312f having a progressively larger length. In the assembled frame 302, the struts 308 form a plurality of closed cells arranged in a plurality of circumferentially extending rows of cells with the cells becoming progressively larger from the inflow end 324 to the outflow end 326. In the illustrated embodiment, each strut 308 has seven apertures 310 defining six segments 312 and five rows of cells, including a first row of cells 328, a second row of cells 330, a third row of cells 332, a fourth row of cells 334, and a fifth row of cells 336, with the cells 328 being the smallest, and each row of cells becoming progressively larger from the inflow end to the outflow end.

The varying lengths of the struts also form angles 338, 340, 342, 346, 348 between pivotably connected struts, wherein the angles progressively decrease from the inflow end 324 to the outflow end 326.

In alternative embodiments, one or more segments can have unequal lengths and one or more segments can have equal lengths. For example, the segment 312a can be the shortest segment, segments 312b, 312c, 312d, 312e can have equal lengths, and the segment 312f can be the longest segment. In still other embodiments, apertures 310 can be equally spaced along the length of each strut, forming segments of equal lengths.

As shown in FIG. 5, each strut 308 can be curved helically with respect to the longitudinal axis A of the frame to define an annular shape of the frame 302. The helical curve provides each strut with a concave, radial inner surface (the surface facing the longitudinal axis A) and an opposing convex, radial outer surface (the surface facing away from the longitudinal axis A).

Apertures 310 can be used to connect the struts 308 to one another using fasteners, such as fasteners 214 as described above.

Each strut 308 can be curved or arranged such that it is concave with respect to the outflow end 326 of the frame 302.

In some embodiments, due to the elasticity of the struts 308 and the connections between overlapping struts, the degree of curvature of a strut can change during radial compression and expansion of the frame.

As with prosthetic valve 200, in the expanded configuration, the curvature of the struts 308 can give the frame 302 a non-cylindrical, tapered shape (e.g., a frustoconical shape, a V-shape, or a Y-shape) wherein the outflow end 326 has a first diameter D1 larger than a second diameter D2 of the inflow end 324. This configuration can reduce the pressure gradient across the prosthetic valve 300 and improve hemodynamics.

In particular embodiments, the draft angle between lines A and C in frame 302 can be between 2 and 15 degrees. In particular embodiments, the draft angle can be at least 2 degrees, at least 5 degrees, at least 10 degrees, at least 20 degrees, at least 30 degrees, at least 40 degrees, or at least 50 degrees. In particular embodiments, the ratio of the outflow diameter D1 to the inflow diameter D2 is at least greater than 1, at least greater than 1.1, at least greater than 1.2, at least greater than 1.4, or at least greater than 1.5.

In some embodiments, there is a 2-3 mm difference between the outflow diameter D1 and the inflow diameter D2. In one specific example, the outflow diameter D1 is about 30 mm and the inflow diameter D2 is about 27 mm. In another example, the outflow diameter D1 is about 31.5 mm and the inflow diameter D2 is about 29 mm. In another example, the outflow diameter D1 is about 24.5 mm and the inflow diameter D2 is about 22 mm.

Further details regarding prosthetic valves having frames with a tapered shape can be found in International Application Pub. No. WO2021086933A2, filed October 18, 2019, and U.S. Patent Application Pub. No. US2020/0188099A1, filed February 11, 2020.

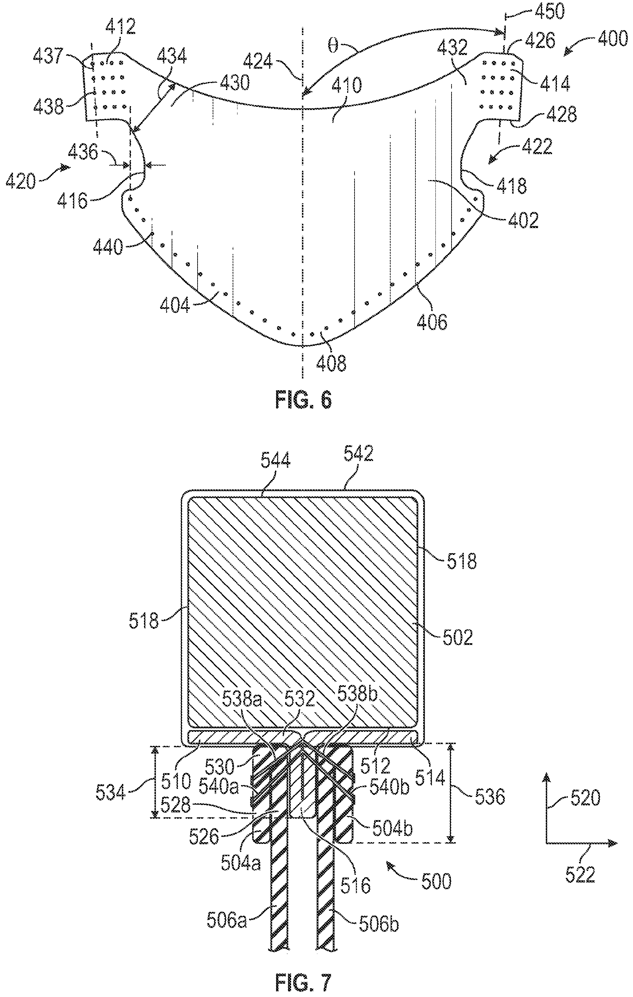

Turning now to FIG. 6, an exemplary leaflet 400 that may be included in a valvular structure of a prosthetic heart valve (e.g., valvular structure 18 of prosthetic heart valve 10 of FIG. 1) is shown. The leaflet 400 is laid flat for the purpose of illustration. The leaflet 400 can comprise a main body (or body) 402 with cusp (inflow) edge portions 404 and 406. In particular the cusp edge portions 404 and 406 may include a first (left) side cusp edge portion 404 and a second (right) side cusp edge portion 406 which connect together at an inflow-most end portion 408 of the leaflet 400. Together, the first and second cusp edge portions 404 and 406 form a scallop line of the leaflet 400.

The leaflet 400 can include commissure tabs 412 and 414, extending from opposite sides of the main body 402. The commissure tabs can be configured for engagement with corresponding commissure tabs of adjacent leaflets to form commissures, and for attachment to the frame of the prosthetic heart valve (e.g., through an attachment member of the commissure, as described further herein). An outflow edge portion 410 extends across the leaflet 400, between the commissure tabs 412 and 414.

A curved edge portion 416, 418 extends between and connects a corresponding one of the commissure tabs 412 and 414 and a corresponding one of the first and second cusp edge portions 404 and 406. Each curved edge portion 416, 418 defines an open region on either side of the leaflet 400, referred to herein as a window 420, 422.

As shown in FIG. 6, each of the first and second cusp edge portions 404 and 406 are angled from a bottom edge of a corresponding one of the curved edge portions 416 and 418 to the inflow end portion 408. An upper edge of each of the first and second cusp edge portions 404 and 406 is arranged inward of an outer edge of a corresponding one of the commissures tabs 412 and 414, in a lateral direction that is arranged perpendicular to an axial direction, the axial direction running from the outflow edge portion 410 to the inflow end portion 408, in parallel to a centerline 424 of the leaflet. As a result, the overall shape of the leaflet is tapered from the outflow edge portion 410 to the inflow end portion 408.

In some embodiments, a shape of the leaflet 400 can be configured such that the commissure tabs 412 and 414 are angled relative to the axial direction which is parallel with the centerline 424 extending through a center of the leaflet 400, between the outflow edge portion 410 and the inflow end portion 408. For example, as shown in FIG. 6, lateral (upper and lower) edges 426 and 428 of each commissure tab 412, 414 can be angled, at an angle θ, from vertical (e.g., centerline 424). As shown in FIG. 6, the angle θ can be defined between the centerline 424 and line 450 which extends through the commissure tabs 412, 414, perpendicular to the lateral edges 426 and 428.

In some embodiments, the angle θ can be selected to match or be similar to (e.g., within a selected, finite range of) the draft angle of the frame of the prosthetic heart valve. For example, as described above with reference to FIGS. 4 and 5, the frame may be tapered from the outflow end to the inflow end of the frame, creating a tapered shape of the frame defined by the draft angle (angle between lines A and C in FIGS. 4 and 5). By selecting the angle θ to match or be close to matching (e.g., within 5-10% or within 5% of) the draft angle of the frame, stresses concentrated at the commissure tabs of the commissure may be reduced, while also allowing sufficient opening of the leaflet during operation of the valve, *in vivo* (e.g., during opening and closing of the leaflet).

In some embodiments, the angle θ can be selected based on the draft angle of the frame and/or based on the geometry of the leaflet, in order to reduce stresses experienced at the commissure while also allowing sufficient opening of the leaflet during valve operation, *in vivo.* In some embodiments, a tapered frame may facilitate a more cylindrical opening of the leaflets, while maintaining the distance between leaflets and the frame during the open phase (e.g., to reduce or avoid abrasion).

In some embodiments, dimensions of the windows 420 and 422 and/or neck regions 430 and 432 of the leaflet 400 can be selected to maximize a width of the leaflet 400 at each neck region 430, 432, while also minimizing a surface area of each window 420, 422. Each neck region 430, 432 is defined between a corresponding one of the commissure tabs 412 and 414 and the main body 402 of the leaflet 400. For example, in some embodiments, a width 434 of each neck region 430 and 432 and a window width 436 of each window 420 and 422 may be selected to help with leaflet stress distribution during the cyclic loading and unloading (e.g., during closing and opening of the leaflet assembly during valve operation) and decrease tissue strain on the leaflet, in order to increase longevity of the leaflet 400.

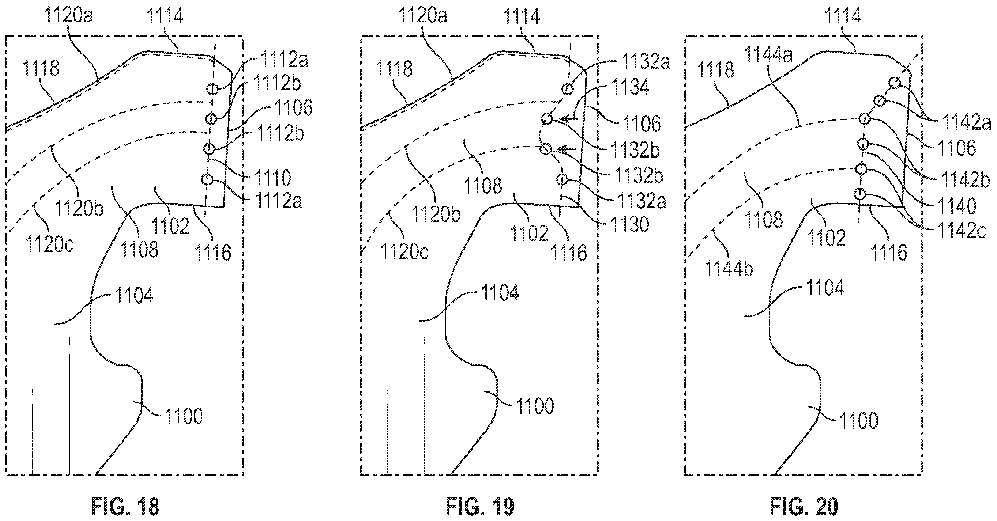

In some embodiments, the commissure tabs 412 and 414 can include a plurality of lines of apertures 438, adapted to receive lines of fasteners (e.g., sutures) during folding and securing of the commissure tabs to form commissures (as described further below with reference to FIGS. 7-9, 14, and 15). These lines of apertures 438 may be referred to herein as stitching lines 437. As shown in FIG. 6, each commissure tab 412 and 414 includes four relatively straight stitching lines 437. However, in alternate embodiments, each commissure tab 412 and 414 can include more or less than four stitching lines 437 (e.g., one, two, three, five, or the like). Further, in some embodiments, instead of a relatively straight stitching line (e.g., with all apertures 438 of the same stitching line arranged in a relatively straight line), one or more of the stitching lines 437 may be non-straight with at least a portion (e.g., one or more apertures 438) that is offset from a remaining portion of the stitching line 437. Examples of leaflets having non-straight stitching lines with offset apertures are shown in FIGS. 19 and 20, as described further below.

Returning in FIG. 6, in some embodiments, each of the first and second cusp edge portions 404 and 406 can include a line of apertures 440 adapted to receive a line of fasteners (e.g., sutures) which secures the first and second cusp edge portions 404 and 406 to a skirt that is configured to be attached to the frame of the prosthetic heart valve. In some embodiments, the skirt may extend from the line of apertures 440 or just above the line of apertures 440 and below (past) a bottom (inflow) edge of the first and second cusp edge portions 404 and 406.

In some embodiments, the skirt can be formed from any of various suitable biocompatible materials, including any of various synthetic materials (e.g., PET) or natural tissue (e.g., pericardial tissue). The skirt can be attached to an inner surface of struts of the frame of the prosthetic heart valve.

A commissure tab of a leaflet, such as one of commissure tabs 412 and 414 of leaflet 400, can be folded, paired with a folded commissure tab of an adjacent leaflet of the valve leaflet assembly to form a commissure, and attached to a commissure support portion of a frame or to a commissure support element configured to be coupled to the commissure support portion (as described further below), thereby forming a commissure. In some embodiments, the commissure tab can be folding vertically, in the axial direction (e.g., about an axis that is perpendicular to the central longitudinal axis of the frame). However, this may not provide enough surface area for attaching the commissure to the commissure support portion of the frame, thereby resulting in a less robust commissure that may become displaced during valve operation. Further, in some embodiments, the commissure tabs can be wrapped around a side and/or back portion of the commissure support portion of the frame and then suture around and to the commissure support portion. However, the increased amount of leaflet material on a side or back (outer-facing surface) of the commissure support portion of the frame may result in increased stresses on the commissure and/or leaflets.

Alternatively, as described further below with reference to FIGS. 7-16, commissures of a prosthetic heart valve can be formed by folding each commissure tab of each leaflet horizontally (e.g., about an axis that is parallel to the central longitudinal axis of the frame, such that folded portion of the folded commissure tabs extend in a circumferential and/or radial direction relative to the central longitudinal axis) and attaching each folded commissure tab to an attachment member which can be arranged on a radially inward-facing surface (inner surface) of the commissure support portion or commissure support element. The attachment member can be attached directly to the commissure support portion or commissure support element, while the folded commissure tabs of the commissure remain positioned radially inward of the commissure support portion of the frame. By folding the commissure tabs radially inward, in a direction perpendicular to the axial direction (e.g., horizontally instead of vertically), a larger surface area is provided for attaching the commissure tab to the attachment member, thereby providing increased support when attaching the attachment member to the commissure support portion of the frame or the commissure support element. As explained further below, by utilizing a fabric attachment member to indirectly attach the commissure tabs of the commissures to the commissure support portions of the frame, direct loading of the leaflets and abrasion of the leaflets against a more rigid surface can be reduced.

FIGS. 7-9, 14, and 15 show exemplary commissure tab assemblies, folded about an axis that is arranged parallel to a central longitudinal axis of the frame of the valve, and attached to an attachment member, as introduced above. These figures further show attachments of these commissure tab assemblies to a commissure support portion of a frame of a prosthetic valve, such as a commissure post or other support structure of the frame of the prosthetic valve (e.g., an actuator or an expansion and locking mechanism), or to a commissure support element configured to be coupled to the commissure support portion of the frame.

In a first embodiment, as illustrated in FIGS. 7-9, the folded commissure tab assembly can be secured together to form a commissure 500 which can then be attached (secured) directly to a commissure support portion of the frame of the prosthetic heart valve, through the attachment member of the commissure tab assembly (or commissure). The commissure support portion can be a rigid support post 502 of the frame. In some embodiments, the support post 502 can be all or a portion of one of an actuator of the frame (e.g., actuator 80 of FIG. 1), an expansion and locking mechanism of the frame (e.g., expansion and locking mechanism 150 of FIGS. 2-3), or another support post of the frame. The frame of the prosthetic heart valve can be one of the frames disclosed above with reference to FIGS. 1-5, and in some embodiments, can combine features of one or more of the frames shown in FIGS. 1-5.

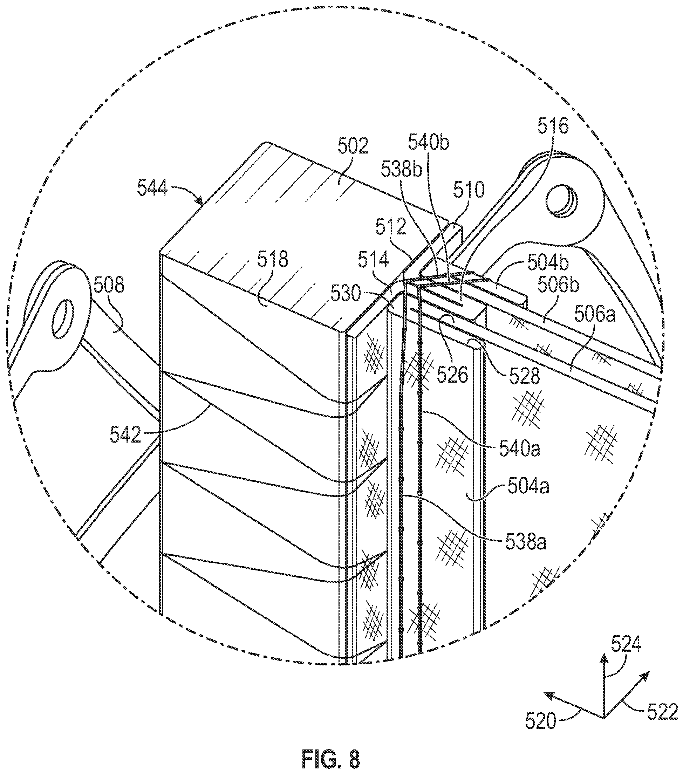

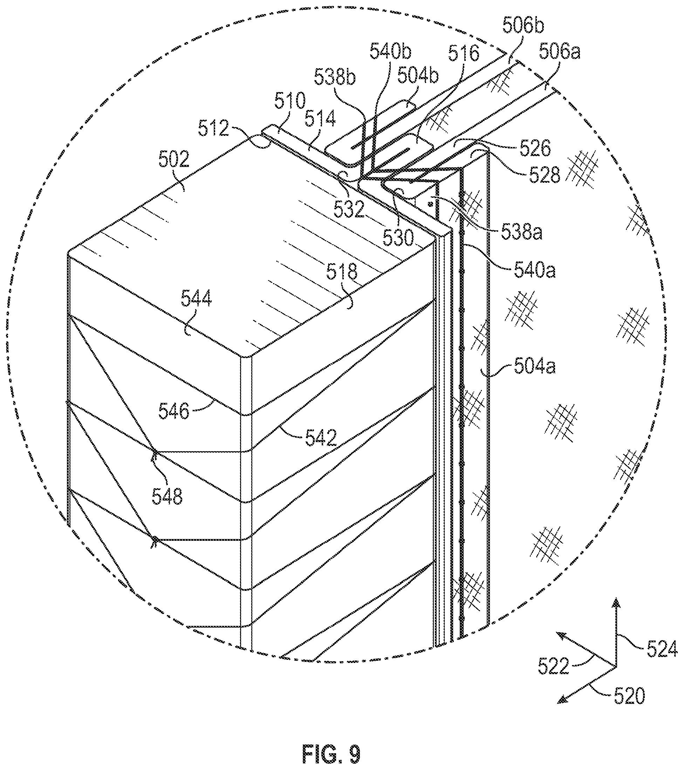

FIG. 7 is a cross-sectional, top view of the commissure 500, attached to the support post 502 of the frame 508 of the prosthetic heart valve. FIGS. 8 and 9 are perspective views of an inner side and outer side of the support post, respectively. In FIG. 9, a remainder of the frame is removed for the purpose of illustration. In FIGS. 7-9, a radial direction 520, lateral (or circumferential) direction 522, and axial direction 524 are shown for reference, where these directions are relative to a central longitudinal axis of the frame of the prosthetic heart valve. For example, a radially inward-facing (inner) surface of a component may face the central longitudinal axis while a radially outward-facing (e.g., outer) surface of a component may face away from the central longitudinal axis. Further, a radially outward-facing surface of a component may be positioned further radially outward from the central longitudinal axis of the frame than an opposite, radially inward-facing surface of the component.

The commissure 500 includes a first commissure tab 504a of a first leaflet 506a and a second commissure tab 504b of a second leaflet 506b. In some embodiments the first and second leaflets 506a and 506b can be the same or similar to the leaflet 400 of FIG. 4. In alternate embodiments, the first and second leaflets 506a and 506b can have a different configuration than that shown in FIG. 4. However, each of the first and second leaflets 506a and 506b has two commissure tabs arranged on opposing sides of a main body of the first or second leaflet.

To form the commissure 500, each of the first commissure tab 504a and second commissure tab 504b is folded over itself to form two commissure tab portions, each extending in the radial direction 520. Each of the folded first commissure tab 504a and the folded second commissure tab 504b is secured to an attachment member 510 (also may be referred to as a reinforcement member or a support member) which is arranged against an inner surface 512 (radially inward-facing, in the radial direction 520 and relative to the central longitudinal axis of the frame of the valve) of the support post 502 and secured to the support post 502.

In some embodiments, the attachment member 510 can be a flexible cloth/fabric comprising one or more layers of cloth/fabric. In some embodiments, the attachment member 510 comprise a synthetic material, such as a polyethylene terephthalate (PET) fabric. In alternate embodiments, the attachment member 510 can comprise a flexible polymeric material.

As shown in FIGS. 7-9, in some embodiments, the attachment member 510 can include a first (base) portion 514 that is positioned against (e.g., in face-sharing contact with) and extends along, in the lateral direction 522, the inner surface 512 of the support post 502. In some embodiments, as shown in FIGS. 7-9, the first portion 514 can only extend along the inner surface 512 and may not extend along additional surfaces of the support post 502. In alternative embodiments, the first portion 514 can additionally extend along at least a portion of lateral (side) surfaces 518 of the support post 502 (but not to the outer surface of the support post 502).

The attachment member 510 can further include a protruding, second portion 516 that protrudes (extends) radially inward toward the central longitudinal axis of the frame and away from the first portion 514. For example, the second portion 516 can extend radially outward from a central region of the first portion 514.

In some embodiments, as shown in FIGS. 7-9, the second portion 516 can include two overlapping layers of the attachment member 510, each extending from a different end of the first portion 514. The two overlapping layers of the second portion 516 of the attachment member 510 can overlap in the lateral direction 522 and each can extend in the radial direction 520. In some embodiments, the second portion 516 can be twice the thickness of the first portion 514, due to it including double the number of layers of the first portion 514 (and the first portion 514 may include one or more layers of fabric, in some embodiments). In this way, the second portion 516 can be thicker than the first portion 514.

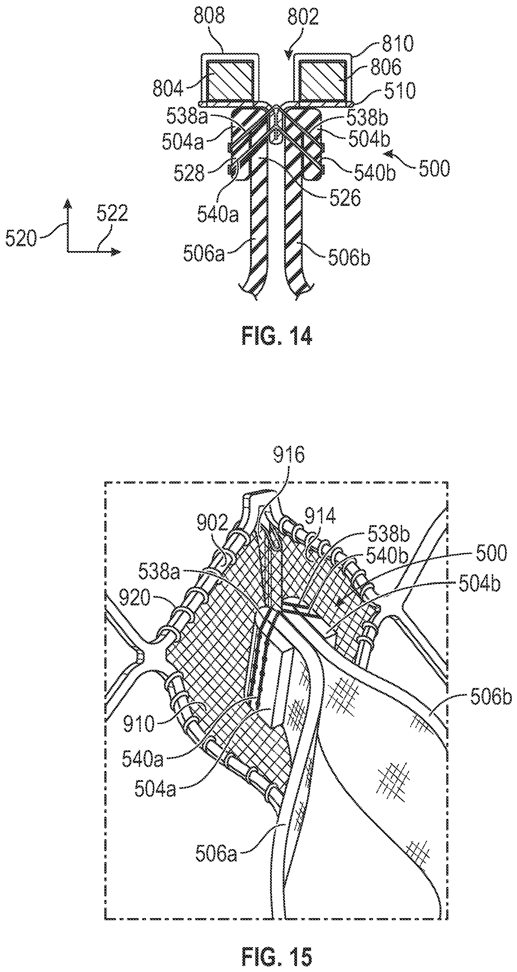

It should be noted that a thickness of the attachment member in FIGS. 7-9 (and FIGS. 14 and 15) relative to the thickness of the leaflets may be exaggerated for the purpose of illustration. Thus, in some embodiments, the attachment member 510 may be thinner than shown in the figures, and thus, the commissure tabs of the leaflets may be arranged closer together, in the lateral direction 522.

As introduced above, each of the first commissure tab 504a and second commissure tab 504b includes two overlapping commissure tab portions, including a first tab portion 526 and a second tab portion 528. Each of the first tab portion 526 and the second tab portion 528 extend in the radial direction 520 and they overlap one another in the lateral direction 522. As shown in FIGS. 7-9, the first tab portion 526 extends from a main body of a corresponding one of the leaflets 506a and 506b, radially outward toward the first portion 514 of the attachment member 510. Each commissure tab 504a, 504b is then folded over itself, from the first tab portion 526, to form the second tab portion 528. The second tab portion extends radially inward, away from the first portion 514 of the attachment member and toward the central longitudinal axis of the frame 508.

As shown in FIGS. 7-9, the first tab portion 526 of the first commissure tab 504a is arranged against (e.g., in face-sharing contact with) a first lateral side of the protruding, second portion 516 of the attachment member 510 and the first tab portion 526 of the second commissure tab 504b is arranged against a second lateral side of the protruding, second portion 516. For example, in some embodiments, a face of the first lateral side of the second portion 516 and an inner face of the first tab portion 526 of the first commissure tab 504a can have face-sharing contact along a height of the first commissure tab 504a (e.g., the height can be a distance between upper and lower edges of the commissure tab, such as upper and lower edges 426 and 428 shown in FIG. 6). Similarly, a face of the second lateral side of the second portion 516 and an inner face of the first tab portion 526 of the second commissure tab 504b can have face-sharing contact along a height of the second commissure tab 504b. In this way, the folded commissure tabs 504a, 504b have a larger surface area for contact with and support from the second portion 516 of the attachment member 510 (as compared to when commissure tabs are folded vertically, in the axial direction, about an axis that is perpendicular to the central longitudinal axis of the valve frame).

Additionally, as shown in FIGS. 7-9, the second tab portion 528 is arranged against the first tab portion 526 and a fold 530 between the first tab portion 526 and the second tab portion 528 is positioned against (e.g., in face-sharing contact with) an inner side of the first portion 514 of the attachment member 510. In this way, each folded commissure tab 504a, 504b can be wedged against the attachment member 510, in a region of a bend 532 between the first portion 514 and the second portion 516. In some embodiments, the bend 532 can be approximately 90 degrees.

The protruding, second portion 516 of the attachment member 510 can extend in the radial direction for a distance 534 (FIG. 7). In some embodiments, the distance 534 (or length of the second portion 516, in the radial direction 520) can be shorter than a length 536 of the second tab portion 528 (e.g., the length of the two overlapping layers of the commissure tabs). In other embodiments, the distance 536 can be the same as the length 536.

The folded first commissure tab 504a and the folded second commissure tab 504b can each be secured to the attachment member 510 via one or more lines of sutures (suture lines). For example, first suture lines 538a and 538b, comprising a plurality of in-and-out stitches, for example, can extend through a corresponding one of the folded commissure tabs 504a and 504b and the attachment member 510 (e.g., at the bend 532). In some embodiments, the first suture lines 538a and 538b can extend through the two overlapping layers of a corresponding one of the folded commissure tabs 504a and 504b and the second portion 516 of the attachment member 510 (through at least one layer of the second portion 516). In some embodiments, second suture lines 540a and 540b, comprising a plurality of in-and-out stitches, for example, can extend through a corresponding one of the folded commissure tabs 504a and 504b (e.g., through their two overlapping layers) and the second portion 516 of the attachment member 510 (e.g., at a radial location inward of the bend 532, such as between the overlapping layers of the second portion 516 of the attachment member 510). In alternate embodiments, there can be more or less suture lines that those shown in FIGS. 7-9 that are configured to secure the folded commissure tabs 504a and 504b to the attachment member 510. As used herein, the term "suture line" can also be referred to as a "stitch line."

In this way, the attachment member 510 and the first and second commissure tabs 504a and 504b are secured together, as explained above, to form the commissure 500.

As shown in FIGS. 7-9, the attachment member 510 can be secured via one or more fasteners to the support post 502 in order to secure the commissure 500 to the support post 502. In this way, the folded first and second commissure tabs 504a and 504b are indirectly attached to the support post 502 (through the attachment member 510). Said another way, the attachment member 510 shields the first and second commissure tabs 504a and 504b (and thus the leaflets 506s and 506b) from coming into contact with the support post 502, since the attachment member 510 is arranged between (in the radial direction 520) the support post 502 and the commissure tabs 504a and 504b.

In some embodiments, the attachment member 510 is secured to the support post 502 via one or more fasteners. For example, as shown in FIGS. 7-9, the attachment member 510 is secured (coupled) to the support post 502 via a suture 542 that can form a plurality of suture loops 546 that cross over on a radially outward-facing (outer) surface 544 of the support post 502, such as disclosed in International Application Pub. No. WO2021/178276 A1. In some embodiments, during assembly, the plurality of loops 546 can be formed by the suture 542, each loop extending around and/or through the attachment member 510 and radially outward from the attachment member 510. The plurality of loops 546 can then be slid over the support post 502 and tightened around the support post 502. In some embodiments, the tightened loops 546 of the suture 542 can be tied off by one or more knots 548.

In this way, by forming a commissure 500 by folding commissure tabs of two adjacent leaflets in the lateral direction and radial direction, about an axis that is arranged parallel with the axial direction 524, such that overlapping tab portions overlap in the lateral direction and extend in the radial direction, a greater surface area is provided for securing the folded commissure tabs to a protruding portion of an attachment member that extends in the radial direction. As a result, a more secure and robust commissure is formed. By utilizing the attachment member and securing the attachment member directly to the support post of the frame, the leaflets may be shielded from coming into contact with the support post and the loading on the leaflets during radial expansion and compression of the frame may be reduced, thereby reducing degradation to the leaflets.

In alternate embodiments, instead of being coupled to a support post of a frame, the commissure 500 can be attached to commissure support portions of different frame elements (e.g., of differently configured frames of different prosthetic heart valves) or to commissure support elements that are configured to be coupled to a commissure support portion of the frame (e.g., an actuator, support post, or the like). In this way, the same commissure 500, as described above with reference to FIGS. 7-9, can be utilized in prosthetic heart valves having different frame designs.

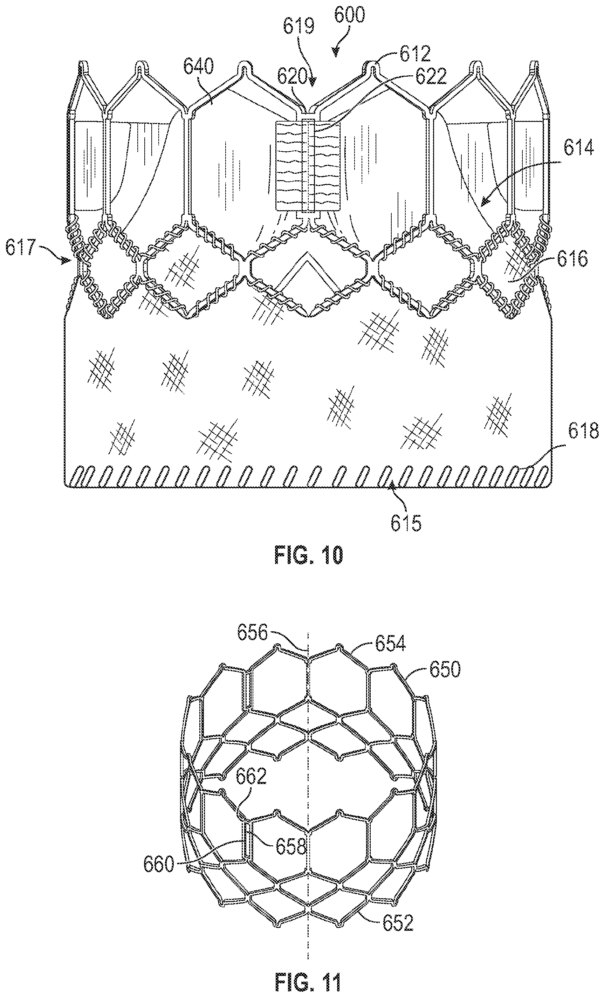

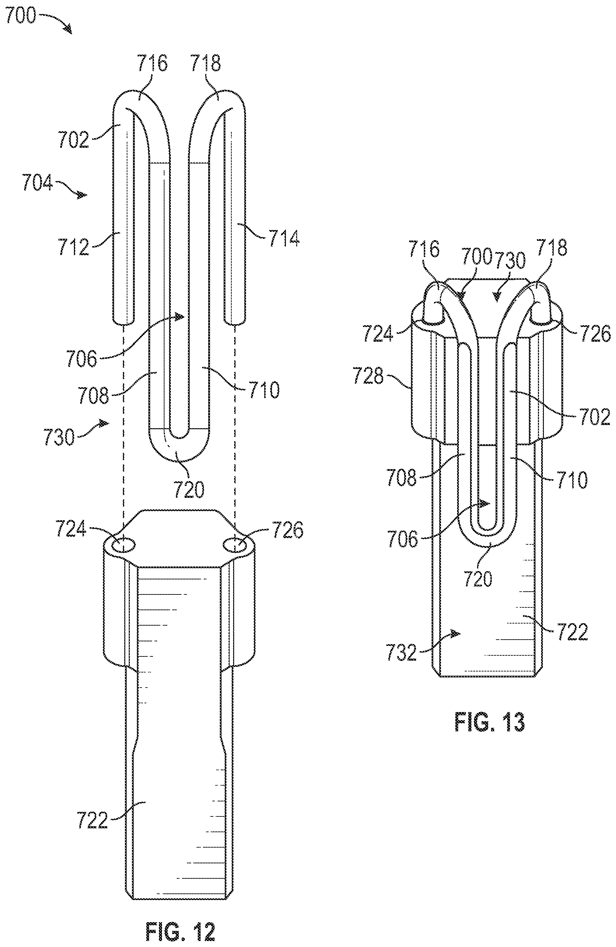

For example, in a second embodiment, as illustrated in FIG. 14, the commissure 500 can be attached directly to a commissure window of a frame of a prosthetic heart valve (FIGS. 10-11) or a (open) commissure window of a commissure support element that is configured to be coupled to a commissure support portion of a support post of the frame (FIGS. 12-13), through the attachment member of the commissure 500.

FIGS. 10 and 11 show exemplary prosthetic heart valve frames with commissure windows integrated therein. Specifically, FIG. 10 shows a prosthetic heart valve 600 including commissure windows incorporated into a frame of the valve, according to an embodiment. The illustrated prosthetic valve is adapted to be implanted in the native aortic annulus, although in other embodiments it can be adapted to be implanted in the other native annuluses of the heart (e.g., the pulmonary, mitral, and tricuspid valves). The prosthetic valve can also be adapted to be implanted in other tubular organs or passageways in the body. The prosthetic valve 600 can have four main components: a stent or frame 612, a valvular structure 614, an inner skirt 616, and a perivalvular outer sealing member or outer skirt 618. The prosthetic valve 600 can have an inflow end portion 615, an intermediate portion 617, and an outflow end portion 619.

The valvular structure 614 can comprise three leaflets 640, collectively forming a leaflet structure, which can be arranged to collapse in a tricuspid arrangement, although in other embodiments there can be greater or fewer number of leaflets (e.g., one or more leaflets 640). The leaflets 640 can be secured to one another at their adjacent sides to form commissures 622 of the leaflet structure 614. In some embodiments the leaflets 640 can have a configuration of leaflet 400, as described above with reference to FIG. 6.

The frame 612 can be formed with a plurality of circumferentially spaced commissure window frame portions 620, that are adapted to mount the commissures 622 of the valvular structure 614 to the frame. For example, each commissure window frame portion 620 can define a commissure window, opening or slot 658, defined by struts 660, 662 of the commissure window, configured to receive commissure tabs of the commissure 622 therein, thereby securing the leaflets to the frame.

The frame 612 can be made of any of various suitable plastically-expandable materials (e.g., stainless steel, etc.) or self-expanding materials (e.g., nickel titanium alloy (NiTi), such as nitinol), as known in the art. When constructed of a plastically-expandable material, the frame 612 (and thus the prosthetic valve 600) can be crimped to a radially collapsed configuration on a delivery catheter and then expanded inside a patient by an inflatable balloon or equivalent expansion mechanism. When constructed of a self-expandable material, the frame 612 (and thus the prosthetic valve 600) can be crimped to a radially collapsed configuration and restrained in the collapsed configuration by insertion into a sheath or equivalent mechanism of a delivery catheter. Once inside the body, the prosthetic valve can be advanced from the delivery sheath, which allows the prosthetic valve to expand to its functional size.

Suitable plastically-expandable materials that can be used to form the frame 612 include, without limitation, stainless steel, a biocompatible, high-strength alloys (e.g., a cobalt-chromium or a nickel-cobalt-chromium alloys), polymers, or combinations thereof. In particular embodiments, frame 612 is made of a nickel-cobalt-chromium-molybdenum alloy, such as MP35N^{®} alloy (SPS Technologies, Jenkintown, Pennsylvania), which is equivalent to UNS R30035 alloy (covered by ASTM F562-02). MP35N^{®} alloy/UNS R30035 alloy comprises 35% nickel, 35% cobalt, 20% chromium, and 10% molybdenum, by weight. Additional details regarding the prosthetic valve 600 and its various components are described in WIPO Patent Application Publication No. WO2018/222799.

FIG. 11 shows a bare frame 650 of a prosthetic heart valve, which, in some embodiments, can be the frame 612 of the prosthetic valve 600 shown in FIG. 10. The frame 650 has an inflow end 652, an outflow end 654, and a central longitudinal axis 656 extending from the inflow end 652 to the outflow end 654 (in an axial direction). The frame 650 can be made of any of the materials described above with reference to FIG. 10. As described above with reference to FIG. 10, the frame 650 can comprise a plurality of commissure windows (open windows) 658 spaced apart from one another, in a circumferential direction, around a circumference of the frame 650. Each commissure window 658 is adapted to receive a pair of commissure tabs of a pair of leaflets arranged into a commissure. As shown in FIG. 11, each commissure window 658 is formed by two axially-extending struts 660, which are spaced apart from one another in the circumferential direction (also referred to as the lateral direction), and two laterally-extending struts 662, which are spaced apart from one another in the axial direction.

In another embodiment, the commissure window configured to receive and be secured to the commissure 500 can be of a separate commissure support element that is configured to be coupled to a support post of the frame. FIGS. 12 and 13 illustrate an embodiment of a commissure support element 700 configured as a unitary wire-form body 702 including a coupling portion 704 (FIG. 12) and a leaflet-receiving window (also referred to as a commissure-receiving portion) 706 defined by axially-extending first and second members 708 and 710. The commissure support element 700 is shown separated from an actuator 722 of a frame of a prosthetic heart valve in FIG. 12 and coupled to the actuator 722 in FIG. 13. In other embodiments, the actuator 722 can instead be an expansion and locking mechanism or a commissure support post, which can be a post that is separate from an actuator coupled to the frame or that is an integral portion of the frame. In particular embodiments, the actuator 722 and the support element 700 can be incorporated in a mechanically expandable prosthetic heart valve, such as those shown in FIGS. 1-5 and described above.

Referring to FIG. 12, the coupling portion 704 can comprise a pair of coupling members 712 and 714. The coupling member 712 can be coupled to the first axial member 708 by a curved portion or member 716, and the coupling member 714 can be coupled to the second axial member 710 by a curved portion or member 718.

The first and second axial members 708 and 710 can be coupled together at their inflow end portions by a member 720. The member 720 can be curved or straight. The members 708, 710, and 720 can at least partially define the leaflet-receiving window 706, which can be open at the top.

As shown in FIG. 13, the commissure support element 700 can be received by or coupled to a post or actuator component 722 of an expandable prosthetic heart valve, which can be configured similarly to any of the post, actuator, or expansion and locking components described herein. The actuator component 722 can comprise a pair of tubular openings or channels 724 and 726 configured to receive the coupling members 712 and 714, respectively. In the illustrated embodiment, the channels 724 and 726 can be located on the sides of the actuator component at the outflow end portion 728 of the actuator component, although the channels may be located anywhere around the perimeter of the actuator component and at any location along its length. The commissure support element 700 can be configured such that when coupled to the actuator component 722, the curved members 716 and 718 extend above (in the outflow direction) an upper surface 730 of the actuator component 722, although the support element may also be disposed elsewhere along the length of the actuator component 722 between the inflow and outflow end portions of the actuator component.

The commissure support element 700 can be formed from a wire-form body, as introduced above, or can be laser cut from a plate or sheet and bent, folded, and/or formed into the specified shape. The commissure support element 700 can comprise a metal material, a polymeric material, and/or combinations or layers thereof.

In alternate embodiments, the commissure support element can have a different shape and/or configuration than shown above, while still having an open, leaflet-receiving window and a coupling portion configured to couple with a support post, actuator, or expansion and locking mechanism of the frame of the prosthetic heart valve. For example, in some embodiments, the coupling portion of the commissure support element may instead be a collar configured to encircle a top of the support post.

In alternate embodiments, instead of having a leaflet-receiving window 706, the commissure support element 700 can include an alternate commissure-receiving portion (which is still coupled to the coupling portion 704 and arranged radially inward of and offset from the coupling portion 704). For example, the commissure-receiving portion may include a relatively planar portion providing a relatively flat surface for being positioned against the attachment member.

As shown in FIG. 14, the commissure 500 can be attached to an open commissure window 802 of a frame of a prosthetic heart valve, such as commissure window 658 of the prosthetic heart valve frames shown in FIGS. 10 and 11, or of a commissure support element, such as leaflet-receiving window 706 of commissure support element 700 of FIGS. 12 and 13. The commissure 500 shown in FIG. 14 may be the same as commissure 500 shown in FIG. 7, except that the attachment member 510 is secured to the commissure window 802 (e.g., instead of the support post 502).

More specifically, as shown in FIG. 14, the commissure window 802 is formed by two axially-extending members 804 and 806 which are spaced apart from one another in the lateral direction 522. In some embodiments, the two axially-extending member 804 and 806 can be axially-extending struts of a commissure window of a frame of a prosthetic heart valve, such as the two-axially-extending struts 660 shown in FIG. 11. In other embodiments, the two axially-extending member 804 and 806 can be axially-extending members of a leaflet-receiving window of a commissure support element configured to be coupled to a commissure support portion of a frame of a prosthetic heat valve, such as the first and second axial members 708 and 718 of commissure support element 700 shown in FIGS. 12 and 13. In other embodiments, the axially-extending members 804 and 806 can be spaced apart struts or posts of an actuator of a mechanically expandable heart valve, such as disclosed in International Application Pub. Nos. WO2021/003167 A1 and WO2021/141878 A1.

The attachment member 510 of the commissure 500 can extend across and between inner sides of the two axially-extending members 804 and 806 (in the lateral direction 522). In some embodiments, ends of the attachment member 510 can curve around and extend along at least a portion of the lateral sides (the sides arranged perpendicular to the inner sides) of the two axially-extending members 804 and 806.

As shown in FIG. 14, the attachment member 510 can be secured to the two axially-extending members 804 and 806 via a first suture 808 and a second suture 810, respectively. In some embodiments a plurality of loops can be formed by the first suture 808 and the second suture 810, and extend around each of the two axially-extending members 804 and 806, similar to as shown in FIGS. 8 and 9 (e.g., for suture 542).

In this way, the base portion 516 of the attachment member 510 can be directly coupled to the two axially-extending members 804 and 806 while the folded commissure tabs are attached to the protruding portion 516 of the attachment member 510, radially inward of the two axially-extending members 804 and 806.

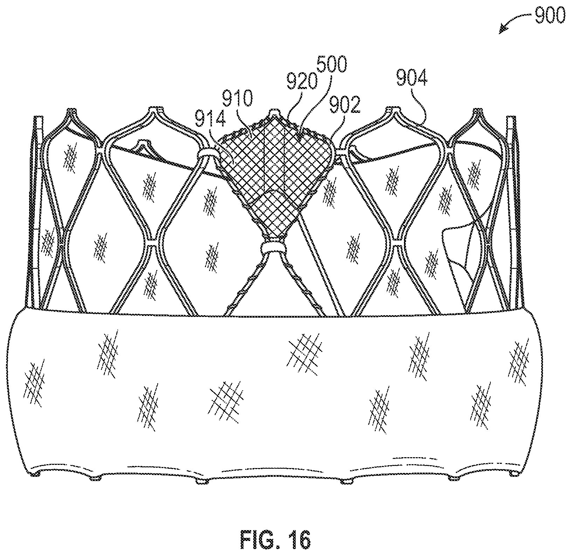

In a third embodiment, as illustrated in FIGS. 15 and 16, the commissure 500 can be attached directly to a cell 902 of a frame 904 of a prosthetic heart valve 900. FIG. 15 shows a portion of an inner side of the frame 904, showing the commissure 500 and an inner side of the attachment member 910 (which may be the same or similar to attachment member 510 of FIGS. 7-9, except that it is arranged across the cell 902 of the frame 904 instead of across a support post of the frame). FIG. 16 shows the prosthetic heart valve 900, showing an outer side of the attachment member 910.

The commissure 500 shown in FIGS. 15 and 16 may be the same as commissure 500 shown in FIG. 7, except that the attachment member 910 extends across the opening of the cell 902 and is secured to the frame struts forming the cell 902.

For example, as shown in FIG. 15, the attachment member 910 includes the base, first portion 914 (similar to first portion 514 in FIGS. 7-9) and a protruding, second portion 916 (similar to second portion 516 shown in FIGS. 7-9). The first portion 914 extends across the opening of the cell 902 and the second portion 916 extends outward, as a fold, from the first portion 914, along a central portion of the cell 902, toward a central longitudinal axis of the frame 904. The first portion 914 of the attachment member 901 can be secured to the struts forming the cell 902 via one or more sutures 920 that extend through the first portion 914 and loop around the frame struts, around a perimeter of the cell 902.

In this way, the base portion 914 of the attachment member 910 can be directly coupled to the struts of the frame while the folded commissure tabs are attached to the protruding portion 916 of the attachment member 910, radially inward of the struts of the frame.

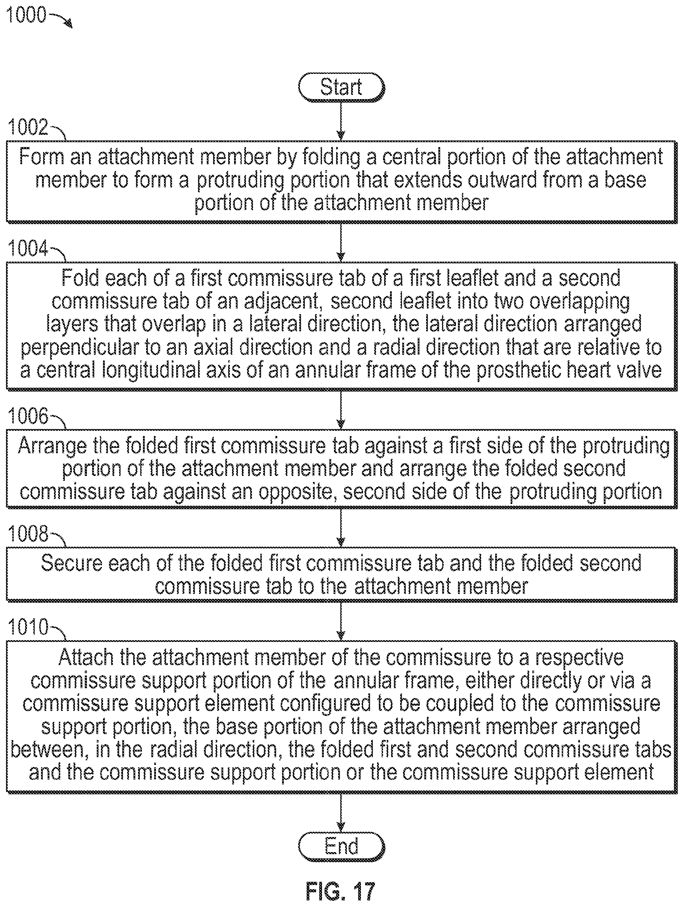

FIG. 17 is flow chart of a method 1000 for assembling a prosthetic heart valve comprising a plurality of leaflets. In some embodiments, the prosthetic heart valve can be one of the valves described herein with reference to FIGS. 1-5, 10, 11, and 16. In particular, method 1000 is a method for forming a plurality of commissures with the plurality of leaflets, each leaflet (e.g., leaflet 400 shown in FIG. 6) including two opposing commissure tabs arranged on opposite sides of a body of the leaflet, and attaching the plurality of commissures to an annular frame of the prosthetic heart valve.

Method 1000 begins at 1002 by forming an attachment member (e.g., attachment member 510 shown in FIGS. 7-9 and 14 or attachment member 910 shown in FIGS. 15 and 16) by folding a central portion of the attachment member to form a protruding portion (e.g., first portion 514 shown in FIGS. 7-9) that extends outward from a base portion (e.g., second portion 516 shown in FIGS. 7-9). In some embodiments, when the base portion of the attachment member is coupled to an inner surface of a commissure support portion of a frame of the prosthetic heart valve (either directly of via a commissure support element), the protruding portion extends in the radial direction, toward the central longitudinal axis of the frame of the valve and away from the base portion. In some embodiments, as explained above with reference to FIGS. 7-9, 14, and 15, the attachment member comprises a fabric material.

At 1004, method 1000 can include folding each of a first commissure tab of a first leaflet and a second commissure tab of an adjacent, second leaflet into two overlapping layers that overlap in a lateral direction, the lateral direction arranged perpendicular to an axial direction and a radial direction that are relative to a central longitudinal axis of an annular frame of the prosthetic heart valve. For example, each of the first commissure tab and the second commissure tab can be folded into two overlapping layers that include a first tab portion (e.g., first tab portion 526 of FIGS. 7-9) and a second tab portion (e.g., second tab portion 528 of FIGS. 7-9). In some embodiments, as explained above with reference to FIGS. 7-9, the first tab portion extends from a main body of a corresponding one of the first and second leaflets, radially outward toward the base (first) portion of the attachment member. Each commissure tab is then folded over itself, from the first tab portion, to form the second tab portion. The second tab portion extends radially inward, away from the base portion of the attachment member and toward the central longitudinal axis of the frame. In some embodiments, as shown in FIG. 7 for example, majority portions of each of the first and second tab portions of each of the first and second commissure tabs can extend in parallel with one another, in the radial direction.

At 1006, method 1000 can include arranging the folded first commissure tab against a first side of the protruding portion of the attachment member and arranging the folded second commissure tab against an opposite, second side of the protruding portion. In some embodiments, the arranging at 1006 can include arranging a face of the first side of the protruding portion and an inner face of the first tab portion of the first commissure tab in face-sharing contact with one another, along a height of the first commissure tab, the height arranged in the axial direction, and arranging a face of the second side of the protruding portion and an inner face of the first tab portion of the second commissure tab in face-sharing contact with one another, along a height of the second commissure tab. In some embodiments, the arranging at 1006 can further include, for each of the first commissure tab and the second commissure tab, arranging a fold between the first tab portion and the second tab portion against an inner side of the base portion of the attachment member such that each of the folded first commissure tab and the folded second commissure tab is wedged against the attachment member, in a region of a bend between the base portion and the protruding portion.

At 1008, method 1000 can include securing each of the (folded) first commissure tab and the (folded) second commissure tab to the attachment member. In some embodiments, the securing can include extending one or more sutures (or suture lines) through the two overlapping layers of the first commissure tab and the protruding portion and extending one or more sutures (or suture lines) through the two overlapping layers of the second commissure tab and the protruding portion. In some embodiments, the protruding portion can include two overlapping layers, each extending from a different end of the base portion, and the securing can include extending one or more sutures (or suture lines) through the two overlapping layers of the first commissure tab and a first layer of the protruding portion and extending one or more sutures (or suture lines) through the two overlapping layers of the second commissure tab and a second layer of the protruding portion.

At 1010, method 1000 can include, for each commissure, attaching the attachment member to a respective commissure support portion of the annular frame, either directly or via a commissure support element configured to be coupled to the commissure support portion, the base portion of the attachment member arranged between, in the radial direction, the folded first and second commissure tabs and the commissure support portion or the commissure support element.

In some embodiments, attaching the attachment member to a respective commissure support portion of the annular frame, either directly or via a commissure support element, includes arranging the base portion of the attachment member against an inner surface of a support post of the annular frame and securing the attachment member to the support post via a suture that extends through the attachment member and around an outer surface of the support post. In some embodiments, the support post is at least a portion of an actuator or an expansion and locking mechanism of the frame.

In other embodiments, attaching the attachment member to a respective commissure support portion of the annular frame, either directly or via a commissure support element, includes arranging the base portion of the attachment member against an inner surface of an open commissure window of the annular frame and securing the attachment member to the open commissure window via at least a first suture that extends through a first end of the base portion of the attachment member and around a first axially-extending strut that forms the open commissure window and a second suture that extends through an opposite, second end of the base portion of the attachment member and around a second axially-extending strut that forms the open commissure window.

In still other embodiments, attaching the attachment member to a respective commissure support portion of the annular frame, either directly or via a commissure support element, includes arranging the base portion of the attachment member against an inner surface of a leaflet-receiving window of the commissure support element, securing the attachment member to the leaflet-receiving window via at least a first suture that extends through a first end of the base portion of the attachment member and around a first axial member forming the leaflet-receiving window and a second suture that extends through an opposite, second end of the base portion of the attachment member and around a second axial member forming the leaflet-receiving window, and coupling the commissure support element to a corresponding commissure support portion of the frame.

In some embodiments, attaching the attachment member to a respective commissure support portion of the annular frame, either directly or via a commissure support element, includes arranging the base portion of the attachment member across a cell of the annular frame, the cell formed by angled struts of the annular frame, and securing the attachment member to the angled struts forming the cell via one or more sutures that extend through the base portion of the attachment member and around the angled struts, around a perimeter of the cell.

Together, the commissure and leaflet configuration described herein may provide a leaflet assembly for a prosthetic heart valve that may experience reduced stresses during operation of the valve (due to cyclic opening and closing of the leaflets) and during radial expansion and/or contraction of the frame of the valve. As a result, a longevity of the leaflet assembly may be increased.

The integrity and longevity of the leaflets and the commissures can be further increased by configuring the stitching line(s) of the commissure tabs to reduce stress concentrations along edges of the leaflets that may be more prone to increased stress, and in some cases, tearing. As discussed herein, in some embodiments, commissures can be attached to a support portion of a frame of a prosthetic heart valve (such as a commissure support post, actuator, expansion and locking mechanism, or the like), either directly or via a commissure support element or attachment member, via one or more sutures that extend through the commissure tabs, along one or more stitching lines of the commissure tab that can include a plurality of apertures (stitching apertures) configured to receive the sutures.

FIGS. 18-20 show a detail view of a portion of an exemplary leaflet 1100 with different embodiments of stitching lines on the commissure tabs 1102 of the leaflet 1100. Only one commissure tab 1102 of the leaflet 1100 is shown in FIGS. 18-20, for ease of illustration. However, leaflet 1100 includes two commissure tabs arranged on either side of a body 1104 of the leaflet 1100. For example, in some embodiments, leaflet 1100 may be configured similarly to leaflet 400 shown in FIG. 6 (as described above).

While each of the embodiments shown in FIGS. 18-20 show a single stitching line, it should be noted that alternate numbers of stitching lines are possible. For example, each commissure tab can include multiple of the stitching line embodiments shown in FIGS. 18-20, such as two, three, or four stitching lines all arranged adjacent to one another from an outer edge 1106 of the commissure tab 1102 toward a neck region 1108 (or body) of the leaflet 1100. For example, the commissure tabs of the leaflet 400 of FIG. 6 each include four stitching lines.

Turning first to FIG. 18, a first embodiment of a stitching line 1110 formed by a plurality of apertures (stitching apertures) 1112a-1112b arranged in the commissure tab 1102 is shown. For example, the apertures include outer apertures 1112a and inner apertures 1112b. The outer apertures 1112a are arranged closer to upper and lower edges of the commissure tab 1102 (e.g., upper edge 1114 and lower edge 1116). The upper edge 1114 can be part of (or connected to) an outflow edge 1118 of the leaflet 1100 (e.g., similar to outflow edge portion 410 shown in FIG. 6). The inner apertures 1112b are arranged further away from the upper and lower edges 1114 and 1116 than the outer apertures 1112a. For example, the inner apertures 1112b can be arranged closer to an interior or center portion of the commissure tab 1102. While the stitching line 1110 is shown with four apertures 1112 in FIG. 18 (and FIG. 19), in alternate embodiments, the stitching line 1110 can include more or less than four apertures 1112 (e.g., two, three, five, six, and the like).

As shown in FIG. 18, the stitching line 1110 extends along a relatively straight line which, in some embodiments, can be approximately parallel to or slightly angled from a centerline of the leaflet, which may be arranged in parallel to a central longitudinal axis of the frame (or in some embodiments, a support post of the frame). Thus, each of the outer apertures 1112a and the inner apertures 1112b are aligned with one another along the relatively straight (and in some embodiments, vertical) stitching line 1110.

As shown in FIGS. 18-20 (and in FIG. 6), the edges of the leaflet 1100 extending inward toward the body 1104 and away from the commissure tab 1102 are angled relative to the stitching line 1110. For example, the outflow edge 1118 of the leaflet 1100 angles and curves downward, in the axial direction, as it travels from the upper edge 1114 of the commissure tab 1102 to a centerline of the leaflet 1100 (e.g., centerline 424 shown in FIG. 6). Due to the angled configuration of the leaflet 1100 relative to the stitching line 1110, the stitching line 1110 can serve as a bending line along which the leaflet bends during operation *in vivo,* during transition between the systolic and diastolic phase, thereby resulting in a non-uniform stress distribution.

In FIG. 18, this non-uniform stress distribution is exemplified by stress lines 1120a-1120c. Specifically, stresses developed along a first stress line 1120a, arranged along the outflow edge 1118 of the leaflet 1100, can be higher than stresses developed along more internal portions of the commissure tab 1102 and body 1104 of the leaflet 1100. For example, stresses experienced along stress lines 1120a-1120c may increase from the third stress line 1120c to the first stress line 1120a, such that the highest amount of stress can be experienced proximate to or at the outflow edge 1118. Thus, since the edge portion of the leaflet, such as the outflow edge 1118, may be more prone to tearing, under some conditions, portions of the outflow edge 1118 can become stressed and degraded and/or may tear over time.

Thus, it may be desirable to provide stitching line configurations at the commissure tabs 1102 that result in higher (e.g., maximal) stress formation along more internal portions of the commissure tab 1102, rather than along the outflow edge 1118. Since the more internal portions of the commissure tab 1102 and leaflet 1100 may be more robust and less prone to tearing, these regions may be able to better handle the increased stresses (as compared to the edge portions of the leaflet 1100).

FIG. 19 shows an embodiment of a stitching line 1130 formed by a plurality of apertures 1132a-1132b (e.g., inner apertures 1132b and outer apertures 1132a, which may be the same or similar to apertures 1112a-1112b, except for their placement on the commissure tab) which is adapted to concentrate higher stresses toward internal portions of the commissure tab 1102 and leaflet 1100, and decrease stresses experienced along the outflow edge 1118 of the leaflet 1100.

For example, as shown in FIG. 19, the stitching line 1130 is not straight and instead can have an offset region (relative to a remainder of the stitching line 1130) at a more central portion (internal or middle region) of the commissure tab 1102. In some embodiments, one or more of the inner apertures 1132b can be offset, radially inward (toward the centerline of the body of the leaflet 1100, as shown by arrows 1134), from the outer apertures 1132a. As a result, a curved stitching line 1130 with an inward peak (the peak arranged away from the outer edge 1106) can be formed. This configuration of the stitching line 1130 can result in higher stresses developed along the mid-portion of the commissure tab 1102 and leaflet 1100, between the upper edge 1114 and the lower edge 1116 and away from the outflow edge 1118 of the leaflet 1100. For example, due to the offset configuration of the stitching line 1130, higher stresses may be experienced along stress lines 1120b and 1120c than at stress line 1120a.

FIG. 20 shows another embodiment of a stitching line 1140 formed by a plurality of apertures 1142a-1142c (e.g., inner apertures 1142b and outer apertures 1142a and 1142c, which may be the same or similar to apertures 1112a-1112b, except for the number of apertures and their placement on the commissure tab) which is adapted to concentrate higher stresses at locations that are spaced away from the outflow edge 1118 of the leaflet 1100 (toward internal portions of the commissure tab 1102 and leaflet 1100).

For example, as shown in FIG. 20, the stitching line 1140 is not straight along all its apertures and instead can have an offset region (e.g., including apertures 1142b and 1142c) which is offset, radially inward (toward the centerline of the body of the leaflet 1100) from the outer apertures 1142a that are arranged proximate to the outflow edge 1118. For example, an upper portion of the stitching line 1140 can angle radially inward from the outer edge 1106 and outflow edge 1118 and then a lower portion of the stitching line 1140 can extend in a relatively straight line, but offset radially from the outer edge 1106. As a result, higher stresses can be concentrated along the mid-portion of the commissure tab 1102 and leaflet 1100, originating at the offset, lower portion of the stitching line 1140 (e.g., at stress line 1144a), at a location that is positioned away from the outflow edge 1118. Thus, lower stresses may be experienced proximate to the outflow edge 1118. In some embodiments, the highest stress may be experienced proximate to and/or along stress line 1144a, while lower stresses are experienced outward (in the axial direction) from stress line 1144a, such as proximate to the outflow edge 1118 and at stress line 1144b.

The stitching line configurations shown in FIGS. 19 and 20 can be employed in the commissure arrangements described herein. For example, in some embodiments, leaflet 400 of FIG. 6 can include multiple stitching lines (arranged adjacent one another on the commissure tabs) having the configuration of stitching line 1130 (FIG. 19) and/or stitching line 1140 (FIG. 20). These offset stitching lines can then be used to secure the folded commissure tabs to the attachment members of the commissure, as described above with reference to FIGS. 7-9 and 14-17. In this way, an integrity and longevity of the commissures and leaflets may be further increased.

In view of the many possible embodiments to which the principles of the disclosed technology may be applied, it should be recognized that the illustrated embodiments are only preferred examples of the disclosed technology and should not be taken as limiting the scope of the claimed subject matter. Rather, the scope of the claimed subject matter is defined by the following claims.

## Claims

1. A prosthetic heart valve (100, 900), comprising:
an annular frame (102, 904) comprising a plurality of commissure support portions (502, 802); and
a plurality of leaflets (400, 506a, 506b) situated within the frame (102, 904), each leaflet (400, 506a, 506b) comprising a body (402) and two opposing commissure tabs (504a, 504b) arranged on opposite sides of the body (402), each commissure tab (504a, 504b) being paired with an adjacent commissure tab (504a, 504b) of an adjacent leaflet (400, 506a, 506b) to form a commissure (500);
wherein each commissure (500) of the prosthetic heart valve (100, 900) comprises:
an attachment member (510, 910) including a first portion (514, 914), the first portion (514, 914) extending across and attached directly to a corresponding commissure support portion (502, 802) of the plurality of commissure support portions (502, 802) or to a commissure support element (700) configured to be coupled to the commissure support portion (502, 802), and a second portion (516, 916) that protrudes radially outward from the first portion (514, 914) and extends radially inward toward a central longitudinal axis of the frame (102, 904);
a first commissure tab (504a) of a first leaflet (400, 506a, 506b) of the plurality of leaflets (400, 506a, 506b) arranged adjacent to a first side of the second portion (516, 916) of the attachment member (510, 910) and secured directly to the attachment member (510, 910); and
a second commissure tab (504b) of a second leaflet (400, 506a, 506b) of the plurality of leaflets (400, 506a, 506b) arranged adjacent to an opposite, second side of the second portion (516, 916) of the attachment member (510, 910) and secured directly to the attachment member (510, 910).

2. The prosthetic heart valve (100, 900) of claim 1, wherein:
a) the first portion (514, 914) of the attachment member (510, 910) is arranged against a radially inward facing surface (512) of the commissure support portion (502, 802) or the commissure support element (700) configured to be coupled to the commissure support portion (502, 802) and wherein the first portion (514, 914) of the attachment member (510, 910) extends across either only the radially inward facing surface (512) of the commissure support portion (502, 802) or the commissure support element (700) or only the radially inward facing surface (512) and a portion of lateral surfaces (518) of the commissure support portion (502, 802) or the commissure support element (700), the lateral surfaces (518) arranged perpendicular to the radially inward facing surface (512); and/or
b) the second portion (516, 916) of the attachment member (510, 910) extends radially outward from a central region of the first portion (514, 914) of the attachment member (510, 910) and includes two overlapping layers, each extending from a different inner end of the first portion (514, 914), wherein the two overlapping layers overlap in the lateral direction (522); and/or
c) the attachment member (510, 910) comprises a fabric material.

3. The prosthetic heart valve (100, 900) of claim 1 or claim 2, wherein the first commissure tab (504a) is folded into two overlapping layers that overlap in a lateral direction (522), the lateral direction (522) arranged perpendicular to an axial direction (524) and a radial direction (520) that are relative to the central longitudinal axis, the two overlapping layers arranged adjacent to the first side of the second portion (516, 916) of the attachment member (510, 910) and secured directly to the attachment member (510, 910) and wherein the second commissure tab (504b) is folded into two overlapping layers that overlap in the lateral direction (522), the two overlapping layers arranged adjacent to the second side of the second portion of the attachment member (510, 910) and secured directly to the attachment member (510, 910),
optionally wherein the two overlapping layers of each of the first commissure tab (504a) and the second commissure tab (504b) include a first tab portion (526) arranged against a respective one of the first side and the second side of the second portion (516, 916) of the attachment member (510, 910) and a second tab portion (528) arranged against the first tab portion (526), the first side and the second side of the second portion (516, 916) and the first tab portion (526) and the second tab portion (528) of each of the first commissure tab (504a) and the second commissure tab (504b) all arranged in parallel with one another.

4. The prosthetic heart valve (100, 900) of claim 3, wherein the folded first commissure tab (504a) is wedged against the attachment member (510, 910), in a region of a first bend (532) between the first portion (514, 914) and the first side of the second portion (516, 916) of the attachment member (510, 910) and wherein the folded second commissure tab (504b) is wedged against the attachment member (510, 910), in a region of a second bend between the first portion (514, 914) and the second side of the second portion (516, 916) of the attachment member (510, 910),
optionally wherein the two overlapping layers of the first commissure tab (504a) are secured to the attachment member (510, 910) by one or more suture lines (538a, 540a) extending through the two overlapping layers and the attachment member (510, 910), proximate to the first bend (532) and wherein the two overlapping layers of the second commissure tab (504b) are secured to the attachment member (510, 910) by one or more suture lines (538b, 540b) extending through the two overlapping layers and the attachment member (510, 910), proximate to the second bend.

5. The prosthetic heart valve (100, 900) of any one of claims 1-4, wherein the second portion (516, 916) of the attachment member (510, 910) extends in the axial direction (524), along an entire height of each of the first commissure tab (504a) and the second commissure tab (504b).

6. The prosthetic heart valve (100, 900) of any one of claims 1-5, wherein the attachment member (510, 910) is attached directly to the commissure support portion (502, 802), wherein the commissure support portion (502, 802) is a support post (502) of the frame (102, 904), and wherein the attachment member (510, 910) is attached directly to the support post (502) via a suture (542) that extends through the attachment member (510, 910) and around an outer surface of the support post (502),
optionally wherein the support post (502) is one of an actuator or an expansion and locking mechanism of the frame (102, 904).

7. The prosthetic heart valve (100, 900) of any one of claims 1-6, wherein the attachment member (510, 910) is:
a) attached directly to the commissure support portion (502, 802) and wherein the commissure support portion (502, 802) is a commissure window (802) formed by spaced apart axially-extending struts (804, 806) of the frame (102, 904); or
b) attached directly to a leaflet-receiving window (706) of the commissure support element (700), the leaflet-receiving window (706) formed by spaced apart axial members (708, 710) of the commissure support element (700), via a first suture that extends through a first end of the first portion (514, 914) of the attachment member (510, 910) and around a first axial member (708) of the spaced apart axial members (708, 710) and a second suture that extends through a second end of the first portion (514, 914) of the attachment member (510, 910) and around a second axial member (710) of the spaced apart axial members (708, 710); or
c) arranged across and attached directly to a cell (902) of the annular frame (102, 904), the cell (902) formed by angled struts of the annular frame (102, 904), via one or more sutures (920) extending through the first portion (514, 914) of the attachment member (510, 910) and around the angled struts forming the cell (902), around a perimeter of the cell (902).

8. The prosthetic heart valve (100, 900) of claim 1,
wherein each commissure support portion (502, 802) includes an inner surface facing a central longitudinal axis of the frame (102, 904) and an oppositely arranged, outer surface;
wherein the first portion (514, 914) comprises a base portion (514, 914) extending across the inner surface of the commissure support portion (502, 802) and the second portion (516, 916) comprises a protruding portion (516, 916) that extends radially outward from a central region of the base portion (514, 914) and extends radially inward toward the central longitudinal axis;
wherein the first commissure tab (504a) is folded into two overlapping layers that overlap in a lateral direction (522), the lateral direction (522) arranged perpendicular to an axial direction (524) and a radial direction (520) that are relative to the central longitudinal axis, the two overlapping layers arranged adjacent to a first side of the protruding portion (516, 916) of the attachment member (510, 910) and secured directly to the attachment member (510, 910); and
the second commissure tab (504b)is folded into two overlapping layers that overlap in the lateral direction (522), the two overlapping layers arranged adjacent to an opposite, second side of the protruding portion (516, 916) of the attachment member (510, 910) and secured directly to the attachment member (510, 910).

9. The prosthetic heart valve (100, 900) of claim 8, wherein a first fold between the two overlapping layers of the first commissure tab (504a) is arranged against the base portion (514, 914), adjacent to a first bend (532) between the base portion (514, 914) and the first side of the protruding portion (516, 916), and wherein a second fold between the two overlapping layers of the second commissure tab (504b) is arranged against the base portion (514, 914), adjacent to a second bend between the base portion (514, 914) and the second side of the protruding portion (516, 916).

10. The prosthetic heart valve (100, 900) of either claim 8 or claim 9, wherein:
a) the protruding portion (516, 916) includes two overlapping layers, each extending from a different inner end of the base portion (514, 914), wherein the two overlapping layers of the protruding portion (516, 916) overlap in the lateral direction (522), and wherein the protruding portion (516, 916)of the attachment member (510, 910) extends in the axial direction (524), along an entire height of each of the first commissure tab (504a) and the second commissure tab (504b); and/or
b) the commissure support portion (502, 802) is one of:
at least a portion of a support post (502) of the frame (102, 904), wherein the support post (502) is one of an actuator, an expansion and locking mechanism, and an alternate support post (502) of the frame (102, 904); or
a commissure window (802) formed by spaced apart axially extending struts (804, 806) of the frame (102, 904).

11. A method (1000) of assembling a prosthetic heart valve comprising a plurality of leaflets, comprising:
forming a plurality of commissures with the plurality of leaflets, each leaflet including two opposing commissure tabs arranged on opposite sides of a body of the leaflet, wherein each commissure is formed by:
folding (1004) each of a first commissure tab of a first leaflet and a second commissure tab of an adjacent, second leaflet into two overlapping layers that overlap in a lateral direction, the lateral direction arranged perpendicular to an axial direction and a radial direction that are relative to a central longitudinal axis of an annular frame of the prosthetic heart valve;
arranging (1006) the folded first commissure tab against a first side of a protruding portion of an attachment member and arranging the folded second commissure tab against an opposite, second side of the protruding portion, the protruding portion extending in the radial direction, away from a base portion of the attachment member;
securing (1008) each of the first commissure tab and the second commissure tab to the attachment member; and
for each commissure, attaching (1010) the attachment member to a respective commissure support portion of the annular frame, either directly or via a commissure support element configured to be coupled to the commissure support portion, the base portion of the attachment member arranged between, in the radial direction, the folded first and second commissure tabs and the commissure support portion or the commissure support element.

12. The method (1000) of claim 11, wherein folding (1004) each of the first commissure tab and the second commissure tab includes folding each of the first commissure tab and the second commissure tab into two overlapping layers that include a first tab portion and a second tab portion, wherein the first tab portion extends from a body of a corresponding one of the first and second leaflets, radially outward toward the base portion of the attachment member and the second portion extends radially inward, away from the base portion of the attachment member and toward the central longitudinal axis of the frame.

13. The method (1000) of claim 12, wherein the arranging (1006) includes arranging a face of the first side of the protruding portion and an inner face of the first tab portion of the first commissure tab in face-sharing contact with one another, along a height of the first commissure tab, the height arranged in the axial direction, and arranging a face of the second side of the protruding portion and an inner face of the first tab portion of the second commissure tab in face-sharing contact with one another, along a height of the second commissure tab,
optionally wherein the arranging (1006) further includes, for each of the first commissure tab and the second commissure tab, arranging a fold between the first tab portion and the second tab portion against an inner side of the base portion of the attachment member such that each of the folded first commissure tab and the folded second commissure tab is wedged against the attachment member, in a region of a bend between the base portion and the protruding portion.

14. The method (1000) of any one of claims 11-13, wherein:
a) the securing (1008) each of the first commissure tab and the second commissure tab to the attachment member includes:
extending one or more sutures through the protruding portion and the two overlapping layers of the first commissure tab, along one or more stitching lines of the first commissure tab that includes at least a middle portion that is offset from a remainder of the stitching line, toward a centerline of the body of the corresponding leaflet, the middle portion arranged away from an outflow edge of the corresponding leaflet; and
extending one or more sutures through the protruding portion and the two overlapping layers of the second commissure tab, along one or more stitching lines of the second commissure tab that includes at least a middle portion that is offset from a remainder of the stitching line, toward a centerline of the body of the corresponding leaflet, the middle portion arranged away from an outflow edge of the corresponding leaflet; or
b) the protruding portion includes two overlapping layers, each extending from a different end of the base portion, and wherein the securing each of the first commissure tab and the second commissure tab to the attachment member includes extending one or more sutures through the two overlapping layers of the first commissure tab and a first layer of the protruding portion and extending one or more sutures through the two overlapping layers of the second commissure tab and a second layer of the protruding portion.

15. The method (1000) of any one of claims 11-14, wherein attaching (1010) the attachment member to a respective commissure support portion (502, 802) of the annular frame, either directly or via a commissure support element, includes arranging the base portion of the attachment member against an inner surface of a support post of the annular frame and securing the attachment member to the support post via a suture that extends through the attachment member and around an outer surface of the support post.

## Patentansprüche

1. Herzklappenprothese (100, 900), umfassend:
einen ringförmigen Rahmen (102, 904), der eine Vielzahl von Kommissurstützabschnitten (502, 802) umfasst; und
eine Vielzahl von Segeln (400, 506a, 506b), die sich innerhalb des Rahmens (102, 904) befinden, wobei jedes Segel (400, 506a, 506b) einen Körper (402) und zwei gegenüberliegende Kommissurlaschen (504a, 504b) umfasst, die auf gegenüberliegenden Seiten des Körpers (402) angeordnet sind, wobei jede Kommissurlasche (504a, 504b) mit einer benachbarten Kommissurlasche (504a, 504b) eines benachbarten Segels (400, 506a, 506b) gepaart ist, um eine Kommissur (500) zu bilden;
wobei jede Kommissur (500) der Herzklappenprothese (100, 900) umfasst:
ein Befestigungselement (510, 910), das einen ersten Abschnitt (514, 914) aufweist, wobei sich der erste Abschnitt (514, 914) über einen entsprechenden Kommissurstützabschnitt (502, 802) der Vielzahl von Kommissurstützabschnitten (502, 802) oder ein Kommissurstützelement (700), das dazu ausgelegt ist, mit dem Kommissurstützabschnitt (502, 802) gekoppelt zu werden, erstreckt und direkt daran befestigt ist, und einen zweiten Abschnitt (516, 916), der von dem ersten Abschnitt (514, 914) radial nach außen vorspringt und sich in Richtung einer zentralen Längsachse des Rahmens (102, 904) radial nach innen erstreckt;
eine erste Kommissurlasche (504a) eines ersten Segels (400, 506a, 506b) der Vielzahl von Segeln (400, 506a, 506b), die neben einer ersten Seite des zweiten Abschnitts (516, 916) des Befestigungselements (510, 910) angeordnet und direkt an dem Befestigungselement (510, 910) gesichert ist; und
eine zweite Kommissurlasche (504b) eines zweiten Segels (400, 506a, 506b) der Vielzahl von Segeln (400, 506a, 506b), die neben einer gegenüberliegenden zweiten Seite des zweiten Abschnitts (516, 916) des Befestigungselements (510, 910) angeordnet und direkt an dem Befestigungselement (510, 910) gesichert ist.

2. Herzklappenprothese (100, 900) nach Anspruch 1, wobei:
a) der erste Abschnitt (514, 914) des Befestigungselements (510, 910) gegen eine radial nach innen weisende Oberfläche (512) des Kommissurstützabschnitts (502, 802) oder des Kommissurstützelements (700), das dazu ausgelegt ist, mit dem Kommissurstützabschnitt (502, 802) gekoppelt zu werden, angeordnet ist und wobei sich der erste Abschnitt (514, 914) des Befestigungselements (510, 910) über entweder nur die radial nach innen weisende Oberfläche (512) des Kommissurstützabschnitts (502, 802) oder des Kommissurstützelements (700) oder nur die radial nach innen weisende Oberfläche (512) und einen Abschnitt der lateralen Oberflächen (518) des Kommissurstützabschnitts (502, 802) oder des Kommissurstützelements (700) erstreckt, wobei die lateralen Oberflächen (518) senkrecht zu der radial nach innen weisenden Oberfläche (512) angeordnet sind; und/oder
b) sich der zweite Abschnitt (516, 916) des Befestigungselements (510, 910) von einem zentralen Bereich des ersten Abschnitts (514, 914) des Befestigungselements (510, 910) radial nach außen erstreckt und zwei überlappende Schichten aufweist, die sich jeweils von einem anderen inneren Ende des ersten Abschnitts (514, 914) erstrecken, wobei die zwei überlappenden Schichten in der lateralen Richtung (522) überlappen; und/oder
c) das Befestigungselement (510, 910) ein Gewebematerial umfasst.

3. Herzklappenprothese (100, 900) nach Anspruch 1 oder Anspruch 2, wobei die erste Kommissurlasche (504a) in zwei überlappende Schichten gefaltet ist, die in eine laterale Richtung (522) überlappen, wobei die laterale Richtung (522) senkrecht zu einer axialen Richtung (524) und einer radialen Richtung (520), die relativ zu der zentralen Längsachse sind, angeordnet ist, wobei die zwei überlappenden Schichten neben der ersten Seite des zweiten Abschnitts (516, 916) des Befestigungselements (510, 910) angeordnet und direkt an dem Befestigungselement (510, 910) gesichert sind und wobei die zweite Kommissurlasche (504b) in zwei überlappende Schichten gefaltet ist, die in der lateralen Richtung (522) überlappen, wobei die zwei überlappenden Schichten neben der zweiten Seite des zweiten Abschnitts des Befestigungselements (510, 910) angeordnet und direkt an dem Befestigungselement (510, 910) gesichert sind,
wobei optional die zwei überlappenden Schichten einer jeden der ersten Kommissurlasche (504a) und der zweiten Kommissurlasche (504b) einen ersten Laschenabschnitt (526) aufweisen, der gegen eine jeweilige der ersten Seite und der zweiten Seite des zweiten Abschnitts (516, 916) des Befestigungselements (510, 910) angeordnet ist, und einen zweiten Laschenabschnitt (528), der gegen den ersten Laschenabschnitt (526) angeordnet ist, wobei die erste Seite und die zweite Seite des zweiten Abschnitts (516, 916) und der erste Laschenabschnitt (526) und der zweite Laschenabschnitt (528) einer jeden der ersten Kommissurlasche (504a) und der zweiten Kommissurlasche (504b) alle parallel zueinander angeordnet sind.

4. Herzklappenprothese (100, 900) nach Anspruch 3, wobei die gefaltete erste Kommissurlasche (504a) in einem Bereich einer ersten Biegung (532) zwischen dem ersten Abschnitt (514, 914) und der ersten Seite des zweiten Abschnitts (516, 916) des Befestigungselements (510, 910) gegen das Befestigungselement (510, 910) verkeilt ist und wobei die gefaltete zweite Kommissurlasche (504b) in einem zweiten Bereich einer zweiten Biegung zwischen dem ersten Abschnitt (514, 914) und der zweiten Seite des zweiten Abschnitts (516, 916) des Befestigungselements (510, 910) gegen das Befestigungselement (510, 910) verkeilt ist,
wobei optional die zwei überlappenden Schichten der ersten Kommissurlasche (504a) durch eine oder mehrere Nahtlinien (538a, 540a), die sich in der Nähe der ersten Biegung (532) durch die zwei überlappenden Schichten und das Befestigungselement (510, 910) erstrecken, an dem Befestigungselement (510, 910) gesichert sind, und wobei die zwei überlappenden Schichten der zweiten Kommissurlasche (504b) durch eine oder mehrere Nahtlinien (538b, 540b), die sich in der Nähe der zweiten Biegung durch die zwei überlappenden Schichten und das Befestigungselement (510, 910) erstrecken, an dem Befestigungselement (510, 910) gesichert sind.

5. Herzklappenprothese (100, 900) nach einem der Ansprüche 1-4, wobei sich der zweite Abschnitt (516, 916) des Befestigungselements (510, 910) entlang einer gesamten Höhe einer jeden der ersten Kommissurlasche (504a) und der zweiten Kommissurlasche (504b) in die axiale Richtung (524) erstreckt.

6. Herzklappenprothese (100, 900) nach einem der Ansprüche 1-5, wobei das Befestigungselement (510, 910) direkt an dem Kommissurstützabschnitt (502, 802) befestigt ist, wobei der Kommissurstützabschnitt (502, 802) ein Stützpfosten (502) des Rahmens (102, 904) ist, und wobei das Befestigungselement (510, 910) direkt über eine Naht (542), die sich durch das Befestigungselement (510, 910) und rund um eine Außenseite des Stützpfostens (502) erstreckt, an dem Stützpfosten (502) befestigt ist,
wobei optional der Stützpfosten (502) eins von einem Betätigungselement oder einem Expansions- und Verriegelungsmechanismus des Rahmens (102, 904) ist.

7. Herzklappenprothese (100, 900) nach einem der Ansprüche 1-6, wobei das Befestigungselement (510, 910) ist:
a) direkt an dem Kommissurstützabschnitt (502, 802) befestigt und wobei der Kommissurstützabschnitt (502, 802) ein Kommissurfenster (802) ist, das durch beabstandete, sich axial erstreckende Streben (804, 806) des Rahmens (102, 904) gebildet ist; oder
b) direkt an einem segelaufnehmenden Fenster (706) des Kommissurstützelements (700) befestigt, wobei das segelaufnehmende Fenster (706) durch beabstandete axiale Elemente (708, 710) des Kommissurstützelements (700) gebildet ist, über eine erste Naht, die sich durch ein erstes Ende des ersten Abschnitts (514, 914) des Befestigungselements (510, 910) und um ein erstes axiales Element (708) der beabstandeten axialen Elemente (708, 710) erstreckt, und eine zweite Naht, die sich durch ein zweites Ende des ersten Abschnitts (514, 914) des Befestigungselements (510, 910) und um ein zweites axiales Element (710) der beabstandeten axialen Elemente (708, 710) erstreckt; oder
c) über einer Zelle (902) des ringförmigen Rahmens (102, 904) angeordnet und direkt daran befestigt, wobei die Zelle (902) durch abgewinkelte Streben des ringförmigen Rahmens (102, 904) gebildet ist, über eine oder mehrere Nähte (920), die sich durch den ersten Abschnitt (514, 914) des Befestigungselements (510, 910) und rund um die die Zelle (902) bildenden abgewinkelten Streben erstrecken, rund um einen Umfang der Zelle (902).

8. Herzklappenprothese (100, 900) nach Anspruch 1, wobei jeder Kommissurstützabschnitt (502, 802) eine Innenfläche, die einer zentralen Längsachse des Rahmens (102, 904) zugewandt ist, und eine gegenüberliegend angeordnete Außenseite aufweist;
wobei der erste Abschnitt (514, 914) einen Basisabschnitt (514, 914) umfasst, der sich über die Innenseite des Kommissurstützabschnitts (502, 802) erstreckt, und der zweite Abschnitt (516, 916) einen vorspringenden Abschnitt (516, 916) umfasst, der sich von einem zentralen Bereich des Basisabschnitts (514, 914) radial nach außen erstreckt und sich in Richtung der zentralen Längsachse radial nach innen erstreckt;
wobei die erste Kommissurlasche (504a) in zwei überlappende Schichten gefaltet ist, die in einer lateralen Richtung (522) überlappen, wobei die laterale Richtung (522) senkrecht zu einer axialen Richtung (524) und einer radialen Richtung (520), die zu der zentralen Längsachse relativ sind, angeordnet ist, wobei die zwei überlappenden Schichten neben einer ersten Seite des vorspringenden Abschnitts (516, 916) des Befestigungselements (510, 910) angeordnet und direkt an dem Befestigungselement (510, 910) gesichert sind; und
die zweite Kommissurlasche (504b) in zwei überlappende Schichten gefaltet ist, die in der lateralen Richtung (522) überlappen, wobei die zwei überlappenden Schichten neben einer gegenüberliegenden zweiten Seite des vorspringenden Abschnitts (516, 916) des Befestigungselements (510, 910) angeordnet und direkt an dem Befestigungselement (510, 910) gesichert sind.

9. Herzklappenprothese (100, 900) nach Anspruch 8, wobei eine erste Falte zwischen den zwei überlappenden Schichten der ersten Kommissurlasche (504a) gegen den Basisabschnitt (514, 914) neben einer ersten Biegung (532) zwischen dem Basisabschnitt (514, 914) und der ersten Seite des vorspringenden Abschnitts (516, 916) angeordnet ist, und wobei eine zweite Falte zwischen den zwei überlappenden Schichten der zweiten Kommissurlasche (504b) gegen den Basisabschnitt (514, 914) neben einer zweiten Biegung zwischen dem Basisabschnitt (514, 914) und der zweiten Seite des vorspringenden Abschnitts (516, 916) angeordnet ist.

10. Herzklappenprothese (100, 900) nach Anspruch 8 oder Anspruch 9, wobei:
a) der vorspringende Abschnitt (516, 916) zwei überlappende Schichten aufweist, die sich jeweils von einem anderen inneren Ende des Basisabschnitts (514, 914) erstrecken, wobei die zwei überlappenden Schichten des vorspringenden Abschnitts (516, 916) in der lateralen Richtung (522) überlappen, und wobei sich der vorspringende Abschnitt (516, 916) des Befestigungselements (510, 910) in der axialen Richtung (524) erstreckt, entlang einer gesamten Höhe einer jeden der ersten Kommissurlasche (504a) und der zweiten Kommissurlasche (504b); und/oder
b) der Kommissurstützabschnitt (502, 802) eins ist von:
mindestens einem Abschnitt eines Stützpfostens (502) des Rahmens (102, 904), wobei der Stützpfosten (502) eins von einem Betätigungselement, einem Expansions- und Verriegelungsmechanismus und einem alternativen Stützpfosten (502) des Rahmens (102, 904) ist; oder
ein Kommissurfenster (802), das durch beabstandete, sich axial erstreckende Streben (804, 806) des Rahmens (102, 904) gebildet ist.

11. Verfahren (1000) zur Montage einer eine Vielzahl von Segeln umfassenden Herzklappenprothese, umfassend:
Bilden einer Vielzahl von Kommissuren mit der Vielzahl von Segeln, wobei jedes Segel zwei gegenüberliegende Kommissurlaschen aufweist, die auf gegenüberliegenden Seiten eines Körpers des Segels angeordnet sind, wobei jede Kommissur gebildet wird durch:
Falten (1004) einer jeden einer ersten Kommissurlasche eines ersten Segels und einer zweiten Kommissurlasche eines benachbarten zweiten Segels in zwei überlappende Schichten, die in einer lateralen Richtung überlappen, wobei die laterale Richtung senkrecht zu einer axialen Richtung und einer radialen Richtung, die relativ zu einer zentralen Längsachse eines ringförmigen Rahmens der Herzklappenprothese sind, angeordnet sind;
Anordnen (1006) der gefalteten ersten Kommissurlasche gegen eine erste Seite eines vorspringenden Abschnitts eines Befestigungselements und Anordnen der gefalteten zweiten Kommissurlasche gegen eine gegenüberliegende zweite Seite des vorspringenden Abschnitts, wobei sich der vorspringende Abschnitt in die radiale Richtung von einem Basisabschnitt des Befestigungselements weg erstreckt;
Sichern (1008) einer jeden der ersten Kommissurlasche und der zweiten Kommissurlasche an dem Befestigungselement; und
für jede Kommissur, Befestigen (1010) des Befestigungselements an einem jeweiligen Kommissurstützabschnitt des ringförmigen Rahmens, entweder direkt oder über ein Kommissurstützelement, das dazu ausgelegt ist, mit dem Kommissurstützabschnitt gekoppelt zu werden, wobei der Basisabschnitt des Befestigungselements in der radialen Richtung zwischen der gefalteten ersten und zweiten Kommissurlasche und dem Kommissurstützabschnitt oder dem Kommissurstützelement angeordnet ist.

12. Verfahren (1000) nach Anspruch 11, wobei das Falten (1004) einer jeden der ersten Kommissurlasche und der zweiten Kommissurlasche das Falten einer jeden der ersten Kommissurlasche und der zweiten Kommissurlasche in zwei überlappende Schichten umfasst, die einen ersten Laschenabschnitt und einen zweiten Laschenabschnitt aufweisen, wobei sich der erste Laschenabschnitt von einem Körper eines entsprechenden einen des ersten und des zweiten Segels radial nach außen in Richtung des Basisabschnitts des Befestigungselements erstreckt und sich der zweite Abschnitt radial nach innen von dem Basisabschnitt des Befestigungselements weg und in Richtung der zentralen Längsachse des Rahmens erstreckt.

13. Verfahren (1000) nach Anspruch 12, wobei das Anordnen (1006) das Anordnen einer Fläche der ersten Seite des vorspringenden Abschnitts und einer Innenfläche des ersten Laschenabschnitts der ersten Kommissurlasche in Flächenkontakt miteinander entlang einer Höhe der ersten Kommissurlasche aufweist, wobei die Höhe in der axialen Richtung angeordnet ist, und das Anordnen einer Fläche der zweiten Seite des vorspringenden Abschnitts und einer Innenfläche des ersten Laschenabschnitts der zweiten Kommissurlasche in Flächenkontakt miteinander, entlang einer Höhe der zweiten Kommissurlasche,
wobei optional das Anordnen (1006) ferner für jede der ersten Kommissurlasche und der zweiten Kommissurlasche das Anordnen einer Falte zwischen dem ersten Laschenabschnitt und dem zweiten Laschenabschnitt gegen eine Innenseite des Basisabschnitts des Befestigungselements aufweist, so dass jede der gefalteten ersten Kommissurlasche und der gefalteten zweiten Kommissurlasche gegen das Befestigungselement in einem Bereich einer Biegung zwischen dem Basisabschnitt und dem vorspringenden Abschnitt verkeilt ist.

14. Verfahren (1000) nach einem der Ansprüche 11-13, wobei:
a) das Sichern (1008) einer jeden der ersten Kommissurlasche und der zweiten Kommissurlasche an dem Befestigungselement aufweist:
Erstrecken einer oder mehrerer Nähte durch den vorspringenden Abschnitt und die zwei überlappenden Schichten der ersten Kommissurlasche entlang einer oder mehrerer Nahtlinien der ersten Kommissurlasche, die mindestens einen mittleren Abschnitt aufweist, der von einem Rest der Nahtlinie in Richtung einer Mittellinie des Körpers des entsprechenden Segels versetzt ist, wobei der mittlere Abschnitt von einem Abflussrand des entsprechenden Segels weg angeordnet ist; und
Erstrecken einer oder mehrerer Nähte durch den vorspringenden Abschnitt und die zwei überlappenden Schichten der zweiten Kommissurlasche entlang einer oder mehrerer Nahtlinien der zweiten Kommissurlasche, die mindestens einen mittleren Abschnitt aufweist, der von einem Rest der Nahtlinie in Richtung einer Mittellinie des Körpers des entsprechenden Segels versetzt ist, wobei der mittlere Abschnitt von einem Abflussrand des entsprechenden Segels weg angeordnet ist; oder
b) der vorspringende Abschnitt zwei überlappende Schichten aufweist, die sich jeweils von einem anderen Ende des Basisabschnitts erstrecken, und wobei das Sichern einer jeden der ersten Kommissurlasche und der zweiten Kommissurlasche an dem Befestigungselement das Erstrecken einer oder mehrerer Nähte durch die zwei überlappenden Schichten der ersten Kommissurlasche und einer ersten Schicht des vorspringenden Abschnitts und das Erstrecken einer oder mehrerer Nähte durch die zwei überlappenden Schichten der zweiten Kommissurlasche und einer zweiten Schicht des vorspringenden Abschnitts aufweist.

15. Verfahren (1000) nach einem der Ansprüche 11-14, wobei das Befestigen (1010) des Befestigungselements an einem jeweiligen Kommissurstützabschnitt (502, 802) des ringförmigen Rahmens entweder direkt oder über ein Kommissurstützelement das Anordnen des Basisabschnitts des Befestigungselements gegen eine Innenfläche eines Stützpfostens des ringförmigen Rahmens und das Sichern des Befestigungselements an dem Stützpfosten über eine Naht aufweist, die sich durch das Befestigungselement und rund um eine Außenfläche des Stützpfostens erstreckt.

## Revendications

1. Valvule cardiaque prothétique (100, 900), comprenant :
un cadre annulaire (102, 904) comprenant une pluralité de parties de support de commissure (502, 802) ; et
une pluralité de feuillets (400, 506a, 506b) situés à l'intérieur du cadre (102, 904), chaque feuillet (400, 506a, 506b) comprenant un corps (402) et deux languettes de commissure opposées (504a, 504b) agencées sur des côtés opposés du corps (402), chaque languette de commissure (504a, 504b) étant appariée avec une languette de commissure adjacente (504a, 504b) d'un feuillet adjacent (400, 506a, 506b) pour former une commissure (500) ;
chaque commissure (500) de la valvule cardiaque prothétique (100, 900) comprenant :
un élément de fixation (510, 910) comprenant une première partie (514, 914), la première partie (514, 914) s'étendant en travers d'une partie de support de commissure correspondante (502, 802) de la pluralité de parties de support de commissure (502, 802) et fixée directement à celle-ci, ou à un élément de support de commissure (700) configuré pour être couplé à la partie de support de commissure (502, 802), et une seconde partie (516, 916) qui fait saillie radialement vers l'extérieur à partir de la première partie (514, 914) et s'étend radialement vers l'intérieur en direction d'un axe longitudinal central du cadre (102, 904) ;
une première languette de commissure (504a) d'un premier feuillet (400, 506a, 506b) de la pluralité de feuillets (400, 506a, 506b) agencée de manière adjacente à un premier côté de la seconde partie (516, 916) de l'élément de fixation (510, 910) et fixée directement à l'élément de fixation (510, 910) ; et
une seconde languette de commissure (504b) d'un second feuillet (400, 506a, 506b) de la pluralité de feuillets (400, 506a, 506b) agencée de manière adjacente à un second côté opposé de la seconde partie (516, 916) de l'élément de fixation (510, 910) et fixée directement à l'élément de fixation (510, 910).

2. Valvule cardiaque prothétique (100, 900) selon la revendication 1,
a) la première partie (514, 914) de l'élément de fixation (510, 910) étant agencée contre une surface orientée radialement vers l'intérieur (512) de la partie de support de commissure (502, 802) ou de l'élément de support de commissure (700) configuré pour être couplé à la partie de support de commissure (502, 802), et la première partie (514, 914) de l'élément de fixation (510, 910) s'étendant en travers soit uniquement de la surface orientée radialement vers l'intérieur (512) de la partie de support de commissure (502, 802) ou de l'élément de support de commissure (700), soit uniquement de la surface orientée radialement vers l'intérieur (512) et d'une partie de surfaces latérales (518) de la partie de support de commissure (502, 802) ou de l'élément de support de commissure (700), les surfaces latérales (518) étant agencées perpendiculairement à la surface orientée radialement vers l'intérieur (512) ; et/ou
b) la seconde partie (516, 916) de l'élément de fixation (510, 910) s'étendant radialement vers l'extérieur à partir d'une région centrale de la première partie (514, 914) de l'élément de fixation (510, 910) et comprenant deux couches superposées, chacune s'étendant à partir d'une extrémité intérieure différente de la première partie (514, 914), les deux couches superposées se chevauchant dans la direction latérale (522) ; et/ou
c) l'élément de fixation (510, 910) comprenant un matériau textile.

3. Valvule cardiaque prothétique (100, 900) selon la revendication 1 ou la revendication 2, la première languette de commissure (504a) étant pliée en deux couches superposées se chevauchant dans une direction latérale (522), la direction latérale (522) étant agencée perpendiculairement à une direction axiale (524) et une direction radiale (520) qui sont relatives à l'axe longitudinal central, les deux couches superposées étant agencées de manière adjacente au premier côté de la seconde partie (516, 916) de l'élément de fixation (510, 910) et fixées directement à l'élément de fixation (510, 910), et la seconde languette de commissure (504b) étant pliée en deux couches superposées se chevauchant dans la direction latérale (522), les deux couches superposées étant agencées de manière adjacente au second côté de la seconde partie de l'élément de fixation (510, 910) et fixées directement à l'élément de fixation (510, 910),
éventuellement, les deux couches superposées de chacune de la première languette de commissure (504a) et de la seconde languette de commissure (504b) comprenant une première partie de languette (526) agencée contre un côté respectif parmi le premier côté et le second côté de la seconde partie (516, 916) de l'élément de fixation (510, 910) et une seconde partie de languette (528) agencée contre la première partie de languette (526), le premier côté et le second côté de la seconde partie (516, 916) et la première partie de languette (526) et la seconde partie de languette (528) de chacune de la première languette de commissure (504a) et de la seconde languette de commissure (504b) étant tous agencés parallèlement les uns aux autres.

4. Valvule cardiaque prothétique (100, 900) selon la revendication 3, la première languette de commissure pliée (504a) étant calée contre l'élément de fixation (510, 910), dans une région d'un premier coude (532) entre la première partie (514, 914) et le premier côté de la seconde partie (516, 916) de l'élément de fixation (510, 910), et la seconde languette de commissure pliée (504b) étant calée contre l'élément de fixation (510, 910), dans une région d'un second coude entre la première partie (514, 914) et le second côté de la seconde partie (516, 916) de l'élément de fixation (510, 910),
éventuellement, les deux couches superposées de la première languette de commissure (504a) étant fixées à l'élément de fixation (510, 910) par une ou plusieurs lignes de suture (538a, 540a) s'étendant à travers les deux couches superposées et l'élément de fixation (510, 910), à proximité du premier coude (532), et les deux couches superposées de la seconde languette de commissure (504b) étant fixées à l'élément de fixation (510, 910) par une ou plusieurs lignes de suture (538b, 540b) s'étendant à travers les deux couches superposées et l'élément de fixation (510, 910), à proximité du second coude.

5. Valvule cardiaque prothétique (100, 900) selon l'une quelconque des revendications 1 à 4, la seconde partie (516, 916) de l'élément de fixation (510, 910) s'étendant dans la direction axiale (524), le long d'une hauteur totale de chacune de la première languette de commissure (504a) et de la seconde languette de commissure (504b).

6. Valvule cardiaque prothétique (100, 900) selon l'une quelconque des revendications 1 à 5, l'élément de fixation (510, 910) étant fixé directement à la partie de support de commissure (502, 802), la partie de support de commissure (502, 802) étant un montant de support (502) du cadre (102, 904), et l'élément de fixation (510, 910) étant fixé directement au montant de support (502) par l'intermédiaire d'une suture (542) qui s'étend à travers l'élément de fixation (510, 910) et autour d'une surface extérieure du montant de support (502),
éventuellement, le montant de support (502) étant l'un parmi un actionneur ou un mécanisme d'expansion et de verrouillage du cadre (102, 904).

7. Valvule cardiaque prothétique (100, 900) selon l'une quelconque des revendications 1 à 6, l'élément de fixation (510, 910) étant :
a) fixé directement à la partie de support de commissure (502, 802), et la partie de support de commissure (502, 802) étant une fenêtre de commissure (802) formée par des entretoises s'étendant axialement (804, 806) espacées du cadre (102, 904) ; ou
b) fixé directement à une fenêtre de réception de feuillet (706) de l'élément de support de commissure (700), la fenêtre de réception de feuillet (706) étant formée par des éléments axiaux espacés (708, 710) de l'élément de support de commissure (700), par l'intermédiaire d'une première suture qui s'étend à travers une première extrémité de la première partie (514, 914) de l'élément de fixation (510, 910) et autour d'un premier élément axial (708) des éléments axiaux espacés (708, 710) et d'une seconde suture qui s'étend à travers une seconde extrémité de la première partie (514, 914) de l'élément de fixation (510, 910) et autour d'un second élément axial (710) des éléments axiaux espacés (708, 710) ; ou
c) agencé en travers d'une cellule (902) du cadre annulaire (102, 904) et fixé directement à celle-ci, la cellule (902) étant formée par des entretoises inclinées du cadre annulaire (102, 904), par l'intermédiaire d'une ou plusieurs sutures (920) s'étendant à travers la première partie (514, 914) de l'élément de fixation (510, 910) et autour des entretoises inclinées formant la cellule (902), autour d'un périmètre de la cellule (902).

8. Valvule cardiaque prothétique (100, 900) selon la revendication 1,
chaque partie de support de commissure (502, 802) comprenant une surface intérieure orientée vers un axe longitudinal central du cadre (102, 904) et une surface extérieure agencée de manière opposée ;
la première partie (514, 914) comprenant une partie de base (514, 914) s'étendant en travers de la surface intérieure de la partie de support de commissure (502, 802) et la seconde partie (516, 916) comprenant une partie saillante (516, 916) qui s'étend radialement vers l'extérieur à partir d'une région centrale de la partie de base (514, 914) et s'étend radialement vers l'intérieur en direction de l'axe longitudinal central ; la première languette de commissure (504a) étant pliée en deux couches superposées se chevauchant dans une direction latérale (522), la direction latérale (522) étant agencée perpendiculairement à une direction axiale (524) et une direction radiale (520) qui sont relatives à l'axe longitudinal central, les deux couches superposées étant agencées de manière adjacente à un premier côté de la partie saillante (516, 916) de l'élément de fixation (510, 910) et fixées directement à l'élément de fixation (510, 910) ; et
la seconde languette de commissure (504b) étant pliée en deux couches superposées se chevauchant dans la direction latérale (522), les deux couches superposées étant agencées de manière adjacente à un second côté opposé de la partie saillante (516, 916) de l'élément de fixation (510, 910) et fixées directement à l'élément de fixation (510, 910).

9. Valvule cardiaque prothétique (100, 900) selon la revendication 8, un premier pli entre les deux couches superposées de la première languette de commissure (504a) étant agencé contre la partie de base (514, 914), de manière adjacente à un premier coude (532) entre la partie de base (514, 914) et le premier côté de la partie saillante (516, 916), et un second pli entre les deux couches superposées de la seconde languette de commissure (504b) étant agencé contre la partie de base (514, 914), de manière adjacente à un second coude entre la partie de base (514, 914) et le second côté de la partie saillante (516, 916).

10. Valvule cardiaque prothétique (100, 900) selon la revendication 8 ou la revendication 9,
a) la partie saillante (516, 916) comprenant deux couches superposées, chacune s'étendant à partir d'une extrémité intérieure différente de la partie de base (514, 914), les deux couches superposées de la partie saillante (516, 916) se chevauchant dans la direction latérale (522), et la partie saillante (516, 916) de l'élément de fixation (510, 910) s'étendant dans la direction axiale (524), le long d'une hauteur totale de chacune de la première languette de commissure (504a) et de la seconde languette de commissure (504b) ; et/ou
b) la partie de support de commissure (502, 802) étant l'une parmi :
au moins une partie d'un montant de support (502) du cadre (102, 904), le montant de support (502) étant l'un parmi un actionneur, un mécanisme d'expansion et de verrouillage, et un montant de support alternatif (502) du cadre (102, 904) ; ou
une fenêtre de commissure (802) formée par des entretoises s'étendant axialement (804, 806) espacées du cadre (102, 904).

11. Procédé (1000) d'assemblage d'une valvule cardiaque prothétique comprenant une pluralité de feuillets, comprenant :
la formation d'une pluralité de commissures avec la pluralité de feuillets, chaque feuillet comprenant deux languettes de commissure opposées agencées sur des côtés opposés d'un corps du feuillet, chaque commissure étant formée par :
le pliage (1004) de chacune d'une première languette de commissure d'un premier feuillet et d'une seconde languette de commissure d'un second feuillet adjacent, en deux couches superposées se chevauchant dans une direction latérale, la direction latérale étant agencée perpendiculairement à une direction axiale et une direction radiale qui sont relatives à un axe longitudinal central d'un cadre annulaire de la valvule cardiaque prothétique ;
l'agencement (1006) de la première languette de commissure pliée contre un premier côté d'une partie saillante d'un élément de fixation et l'agencement de la seconde languette de commissure pliée contre un second côté opposé de la partie saillante, la partie saillante s'étendant dans la direction radiale, à l'écart d'une partie de base de l'élément de fixation ;
la fixation (1008) de chacune de la première languette de commissure et de la seconde languette de commissure à l'élément de fixation ; et
pour chaque commissure, la fixation (1010) de l'élément de fixation à une partie de support de commissure respective du cadre annulaire, soit directement soit par l'intermédiaire d'un élément de support de commissure configuré pour être couplé à la partie de support de commissure, la partie de base de l'élément de fixation étant agencée entre, dans la direction radiale, les première et seconde languettes de commissure pliées et la partie de support de commissure ou l'élément de support de commissure.

12. Procédé (1000) selon la revendication 11, le pliage (1004) de chacune de la première languette de commissure et de la seconde languette de commissure comprenant le pliage de chacune de la première languette de commissure et de la seconde languette de commissure en deux couches superposées qui comprennent une première partie de languette et une seconde partie de languette, la première partie de languette s'étendant à partir d'un corps d'un feuillet correspondant parmi les premier et second feuillets, radialement vers l'extérieur en direction de la partie de base de l'élément de fixation, et la seconde partie s'étendant radialement vers l'intérieur, à l'écart de la partie de base de l'élément de fixation et en direction de l'axe longitudinal central du cadre.

13. Procédé (1000) selon la revendication 12, l'agencement (1006) comprenant l'agencement d'une face du premier côté de la partie saillante et d'une face intérieure de la première partie de languette de la première languette de commissure en contact face contre face l'une avec l'autre, le long d'une hauteur de la première languette de commissure, la hauteur étant agencée dans la direction axiale, et l'agencement d'une face du second côté de la partie saillante et d'une face intérieure de la première partie de languette de la seconde languette de commissure en contact face contre face l'une avec l'autre, le long d'une hauteur de la seconde languette de commissure,
éventuellement, l'agencement (1006) comprenant en outre, pour chacune de la première languette de commissure et de la seconde languette de commissure, l'agencement d'un pli entre la première partie de languette et la seconde partie de languette contre un côté intérieur de la partie de base de l'élément de fixation de sorte que chacune de la première languette de commissure pliée et de la seconde languette de commissure pliée soit calée contre l'élément de fixation, dans une région d'un coude entre la partie de base et la partie saillante.

14. Procédé (1000) selon l'une quelconque des revendications 11 à 13 :
a) la fixation (1008) de chacune de la première languette de commissure et de la seconde languette de commissure à l'élément de fixation comprenant :
l'extension d'une ou plusieurs sutures à travers la partie saillante et les deux couches superposées de la première languette de commissure, le long d'une ou plusieurs lignes de couture de la première languette de commissure qui comprend au moins une partie médiane qui est décalée par rapport à un reste de la ligne de couture, en direction d'une ligne centrale du corps du feuillet correspondant, la partie médiane étant agencée à l'écart d'un bord d'éjection du feuillet correspondant ; et
l'extension d'une ou plusieurs sutures à travers la partie saillante et les deux couches superposées de la seconde languette de commissure, le long d'une ou plusieurs lignes de couture de la seconde languette de commissure qui comprend au moins une partie médiane qui est décalée par rapport à un reste de la ligne de couture, en direction d'une ligne centrale du corps du feuillet correspondant, la partie médiane étant agencée à l'écart d'un bord d'éjection du feuillet correspondant ; ou
b) la partie saillante comprenant deux couches superposées, chacune s'étendant à partir d'une extrémité différente de la partie de base, et la fixation de chacune de la première languette de commissure et de la seconde languette de commissure à l'élément de fixation comprenant l'extension d'une ou plusieurs sutures à travers les deux couches superposées de la première languette de commissure et une première couche de la partie saillante et l'extension d'une ou plusieurs sutures à travers les deux couches superposées de la seconde languette de commissure et une seconde couche de la partie saillante.

15. Procédé (1000) selon l'une quelconque des revendications 11 à 14, la fixation (1010) de l'élément de fixation à une partie de support de commissure respective (502, 802) du cadre annulaire, soit directement soit par l'intermédiaire d'un élément de support de commissure, comprenant l'agencement de la partie de base de l'élément de fixation contre une surface intérieure d'un montant de support du cadre annulaire et la fixation de l'élément de fixation au montant de support par l'intermédiaire d'une suture qui s'étend à travers l'élément de fixation et autour d'une surface extérieure du montant de support.
